# EUROPEAN PATENT SPECIFICATION

(11) **EP 0 492 369 B1**
(45) Date of publication and mention of the grant of the patent: **17.09.1997**
(21) Application number: 91121638.0
(22) Date of filing: 17.12.1991
(51) Int. Cl.: C07K 5/02, A61K 38/55

(54) **Novel amino and nitro containing tricyclic compounds useful as inhibitors of ace**
Neue trizyklische Amino- und Nitro Verbindungen mit ACE-hemmenden Eigenschaften
Nouveaux composés tricycliques avec nitro ou amino groupements, utiles comme inhibiteurs d'ACE

(30) Priority: 21.12.1990 US 633572; 23.10.1991 US 777625
(43) Date of publication of application: 01.07.1992
(73) Proprietor: MERRELL PHARMACEUTICALS INC., Cincinnati, Ohio 45215-6300 (US)
(72) Inventor: Flynn, Gary A., Cincinnati, Ohio 45243 (US); Beight, Douglas W., Cincinnati, Ohio 45246 (US)
(74) Representative: VOSSIUS & PARTNER

(56) References cited:
- EP-A- 0 249 223
- US-A- 4 584 294

## Description

The compounds of the present invention are inhibitors of Angiotensin-Converting Enzyme (ACE). ACE is a peptidyl dipeptidase which catalyzes the conversion of angiotensin I to angiotensin II. Angiotensin II is a powerful vasopressor which also stimulates aldosterone secretion by the adrenal cortex.

Inhibition of ACE lowers levels of angiotensin II and thus inhibits the vasopressor, hypertensive and hyperaldosteronemic effects caused thereby. It is known that inhibition of ACE is useful in the treatment of patients suffering from disease states such as hypertension and chronic congestive heart failure [See William W. Douglas, "Polypeptides - Angiotensin, Plasma Kinins, and Others", Chapter 27, in GOODMAN AND GILLMAN'S THE PHARMACOLOGICAL BASIS OF THERAPEUTICS, 7th edition, 1985, pp. 652-3, MacMillan Publishing Co., New York, New York]. In addition, it has been disclosed that ACE inhibitors are useful in treating cognitive disorders [ DE-OS 3901-291, published August 3, 1989].

Certain tricyclic compounds such as that described by Flynn et al. [*J.Amer.Chem.Soc.* 109, 7914-15 (1987)] and Flynn and Beight [European Patent Application Publication No. 0 249 223 A2, published December 16, 1987] are known as ACE inhibitors. The compounds of the present invention differ from these tricyclic inhibitors of ACE by having an amino or nitro substituent on the aromatic moiety of the tricyclic structure.

It has now been found that the amino or nitro substituted compounds of the present invention possess an unexpectedly prolonged duration of activity in comparison to other ACE inhibitors of similar structure.

The present invention provides novel compounds of the formula (1) 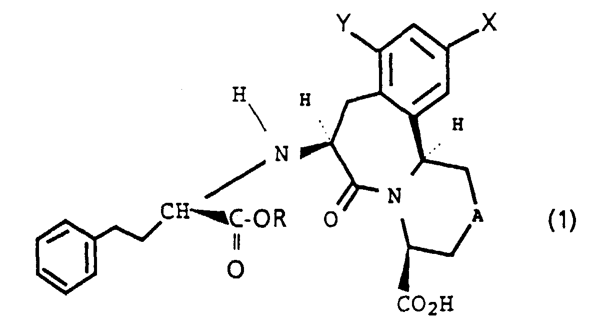 wherein
A is methylene, oxygen, sulfur or N-B wherein B is R₁ or COR₂ wherein R₁ is hydrogen, a C₁-C₄ alkyl or an Ar-Z-group wherein Ar is aryl and Z is a C₀-C₄ alkyl and R₂ is a -CF₃, a C₁-C₁₀ alkyl or an Ar-Z group;
R is hydrogen or a C₁-C₄ alkyl; and
X and Y are each independently hydrogen, nitro or amino, with the proviso that one of X and Y is hydrogen and one of X and Y is other than hydrogen; and
the pharmaceutically acceptable salts thereof.

The present invention further provides pharmaceutical compositions useful in inhibiting ACE which comprise a compound of formula (1).

In addition, the present invention provides a composition comprising an assayable amount of a compound of formula (1) in admixture or otherwise in association with an inert carrier. The present invention also provides a pharmaceutical composition comprising an effective immunosuppressive amount of a compound of formula (1) in admixture or otherwise in association with one or more pharmaceutically acceptable carriers or excipients.

As used herein, the term "C₁-C₄ alkyl" refers to a saturated straight or branched chain hydrocarbyl radical of one to four carbon atoms and includes methyl, ethyl, propyl, isopropyl, n-butyl, isobutyl, tertiary butyl and the like. The term "C₁-C₁₀ alkyl" refers to a saturated straight or branched chain hydrocarbyl radical of one to ten carbon atoms, respectively, including methyl, ethyl, propyl, isopropyl, n-butyl, isobutyl, tertiary butyl, pentyl, isopentyl, hexyl, 2,3-dimethyl-2-butyl, heptyl, 2,2-dimethyl-3-pentyl, 2-methyl-2-hexyl, octyl, 4-methyl-3-heptyl and the like. The term "halogen", "halo", "halide" or "X" refers to a chlorine, bromine, or iodine atom.

As used herein, the term "Ar-Z-" refers to a radical wherein Ar is an aryl group and Z is a C₀-C₄ alkyl. The term "Ar" refers to a phenyl or naphthyl group unsubstituted or substituted with from one to three substituents selected from the group consisting of methylenedioxy, hydroxy, C₀-C₄ alkoxy, fluoro and chloro. The term "C₀-C₄ alkyl" refers to a saturated straight or branched chain hydrocarbyl radical of zero to four carbon atoms and includes a bond, methyl, ethyl, propyl, isopropyl, n-butyl, isobutyl, tertiary butyl and the like. Specifically included within the scope of the term "Ar-Z-" are phenyl, naphthyl, phenylmethyl or benzyl, phenylethyl, p-methoxybenzyl, p-fluorobenzyl and p-chlorobenzyl.

The compounds of formula (1), wherein R is hydrogen can be prepared by utilizing procedures and techniques well known and appreciated by one of ordinary skill in the art. A general synthetic scheme for preparing these compounds is set forth in Scheme A wherein all substituents, unless otherwise indicated, are previously defined. 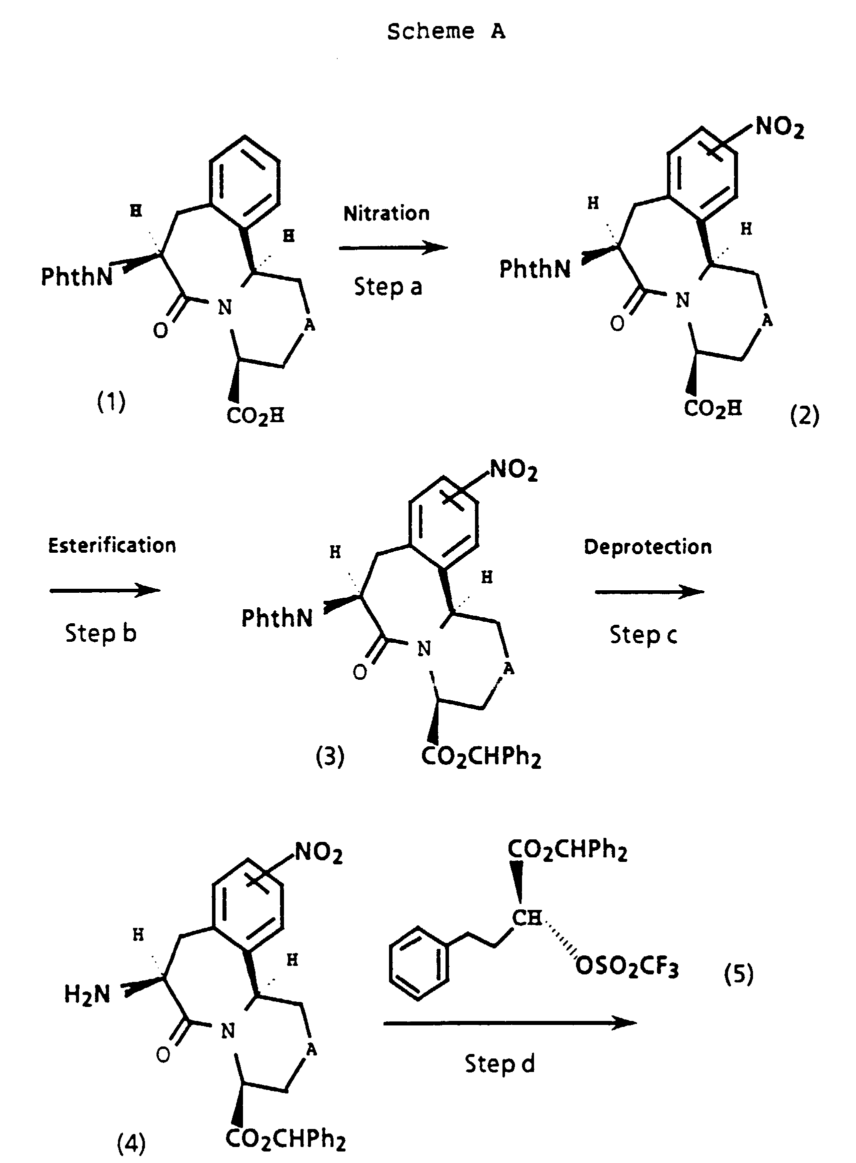 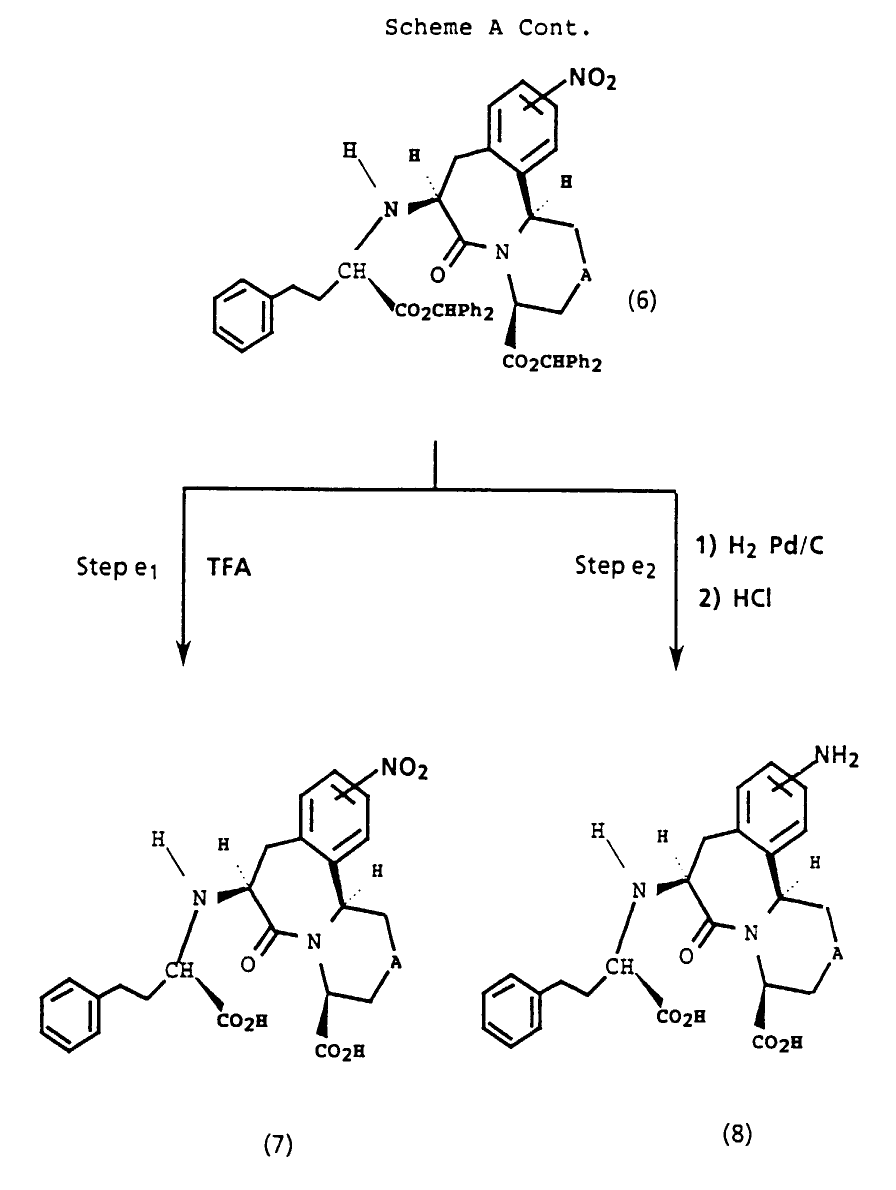 Scheme A provides a general synthetic scheme for preparing compounds of formula (1) wherein R is hydrogen.

In step a, the appropriate phthalimide protected amino/carboxylic acid compound of structure (1) is nitrated to give a mixture of the corresponding 9-nitro and 11-nitro/phthalimide protected amino/carboxylic acid compounds of structure (2).

For example, the appropriate phthalimide protected amino/carboxylic acid compound of structure (1) is contacted with a molar excess of a nitrating agent, such as nitronium tetrafluoroborate. The reactants are typically contacted in a suitable organic solvent, such as methylene chloride. The reactants are typically stirred together for a period of time ranging from 10-50 hours and at a temperature range of from -60°C to 10°C. The 9-nitro and 11-nitro/phthalimide protected amino/carboxylic acid compounds of structure (2) are recovered from the reaction mixture by extractive methods as is known in the art. They can be separated by silica gel chromatography.

In step b, the appropriate individual 9-nitro and 11-nitro/phthalimide protected amino/carboxylic acid compound of structure (2) is esterified to give the corresponding individual 9-nitro and 11-nitro/phthalimide protected amino/carboxylic acid, diphenylmethyl ester compound of structure (3).

For example, the appropriate individual 9-nitro and 11-nitro/phthalimide protected amino/carboxylic acid compound of structure (2) is contacted with a molar equivalent of an appropriate diphenylmethylating agent, such as diphenyldiazomethane. The reactants are typically contacted in a suitable organic solvent, such as methylene chloride. The reactants are typically stirred together at room temperature for a period of time ranging from 1-10 days. The corresponding individual 9-nitro and 11-nitro/phthalimide protected amino/carboxylic acid, diphenylmethyl ester compound of structure (3) is recovered from the reaction zone by evaporation of the solvent. They may be purified by silica gel chromatography.

In step c, the phthalimide protecting group of the appropriate individual 9-nitro and 11-nitro/phthalimide protected amino/carboxylic acid, diphenylmethyl ester compound of structure (3) is removed to give the corresponding individual 9-nitro and 11-nitro/amino/carboxylic acid, diphenylmethyl ester compound of structure (4).

For example, the appropriate individual 9-nitro and 11-nitro/phthalimide protected amino/carboxylic acid, diphenylmethyl ester compound of structure (3) is contacted with a molar excess of hydrazine monohydrate. The reactants are typically contacted in protic organic solvent, such as methanol. The reactants are typically stirred together at room temperature for a period of time ranging from 5-24 hours. The corresponding individual 9-nitro and 11-nitro/amino/carboxylic acid, diphenylmethyl ester compound structure (4) is recovered from the reaction zone by evaporation of the solvent, redissolving in CHCl₃, filtration to remove phthal-hydrazide and removal of the CHCl₃ *in vacuo.*

In step d, the amino functionality of the appropriate individual 9-nitro and 11-nitro/amino/carboxylic acid, diphenylmethyl ester compound of structure (4) is alkylated with (R)-2-trifluoromethanesulfonyl-4-phenylbutyric acid, diphenylmethyl ester (5) to give the corresponding individual 9-nitro and 11-nitro/[1-(carbodiphenylmethoxy)-3-phenyl]propylamino/carboxylic acid, diphenylmethyl ester compound of structure (6).

For example, the appropriate individual 9-nitro and 11-nitro/amino/carboxylic acid, diphenylmethyl ester compound of structure (4) is contacted with a molar excess of (R)-trifluoromethanesulfonyl-4-phenylbutyric acid, diphenylmethyl ester (5) and a molar excess of strong, non-nucleophilic base, such as Proton Sponge. The reactants are typically contacted in a suitable organic solvent such as methylene chloride. The reactants are typically stirred together at room temperature for a period of time ranging from 10-30 hours. The corresponding individual 9-nitro and 11-nitro/[1-(carbodiphenylmethoxy)-3-phenyl]propylamino/carboxylic acid, diphenylmethyl ester compound of structure (6) is recovered from the reaction zone by silica gel chromatography.

In step e₁, the diphenylmethyl ester functionalities of the appropriate individual 9-nitro and 11-nitro/[1-(carbodiphenylmethoxy)-3-phenyl]propylamino/carboxylic acid, diphenylmethyl ester compound of structure (6) are hydrolyzed to give the corresponding individual 9-nitro and 11-nitro/[1-(carboxy)-3-phenyl]propylamino/carboxylic acid compound of structure (7).

For example, the appropriate individual 9-nitro and 11-nitro/[1-(carbodiphenylmethoxy)-3-phenyl]propylamino/carboxylic acid, diphenylmethyl ester compound of structure (6) is contacted with a molar excess of an acid, such as trifluoroacetic acid. The reactants are typically contacted in a suitable organic solvent such as methylene chloride. The reactants are typically stirred together at room temperature for a period of time ranging from 1-24 hours. The corresponding individual 9-nitro and 11-nitro/[1-(carboxy)-3-phenyl]propylamino/carboxylic acid compound of structure (7) is recovered from the reaction zone by removal of solvent and trituration to remove nonpolar by-products followed by reverse phase HPLC where required.

In step e₂, the nitro functionality of the appropriate individual 9-nitro and 11-nitro/[1-(carboxy)-3-phenyl]propylamino/carboxylic acid, diphenylmethyl ester compound of structure (6) is reduced concurrently with diphenylmethyl ester hydrogenolysis to give the corresponding individual 9-amino and 11-amino/(1-(carboxy)-3-phenyl]propylamino/carboxylic acid compound of structure (8).

For example, the individual 9-nitro and 11-nitro/[1-(carboxy)-3-phenyl]propylamino/carboxylic acid, diphenylmethyl ester compound of structure (6) is contacted with a catalytic amount of a hydrogenation catalyst, such as 10% palladium/carbon. The reactants are typically contacted in a suitable solvent mixture such as tetrahydrofuran/water. The reactants are typically shaken under a hydrogen atmosphere of 35-45 psi at room temperature for a period of time ranging from 5-24 hours. The individual 9-amino and 11-amino/[1-(carboxy)-3-phenyl]propylamino/carboxylic acid compound of structure (8) is recovered from the reaction zone by evaporation of the solvent. They may then be converted to their dihydrochloride salts and triturated with hexane to remove the diphenylmethane.

For those compounds of formula (1) wherein A is N-COR₂, the N-COR₂ group of the appropriate individual 9-nitro and 11-nitro/[1-(carboxy)-3-phenyl]propylamino/carboxylic acid compound of structure (7) or the appropriate individual 9-amino and 11-amino/[1-(carboxy)-3-phenyl]propylamino/carboxylic acid compound of structure (8) may be cleaved by techniques and procedures well known and appreciated in the art, such as lithium hydroxide, to give the corresponding compounds of formula (1) wherein A is NH.

The compounds of formula (1), wherein R is a C₁-C₄ alkyl can be prepared by utilizing procedures and techniques well known and appreciated by one of ordinary skill in the art. A general synthetic scheme for preparing these compounds is set forth in Scheme B wherein all substituents, unless otherwise indicated, are previously defined.

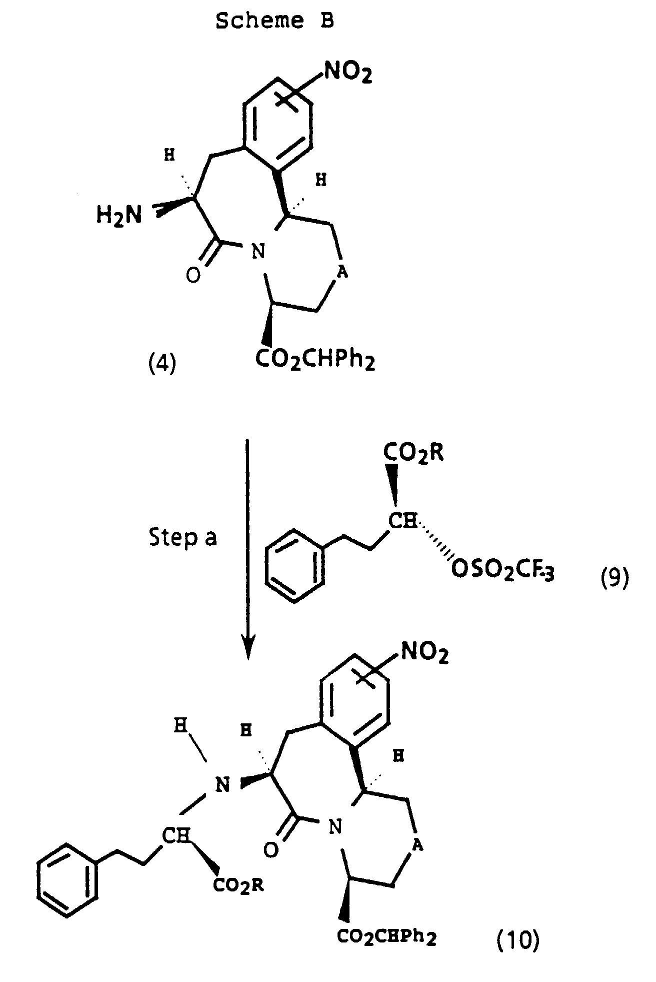

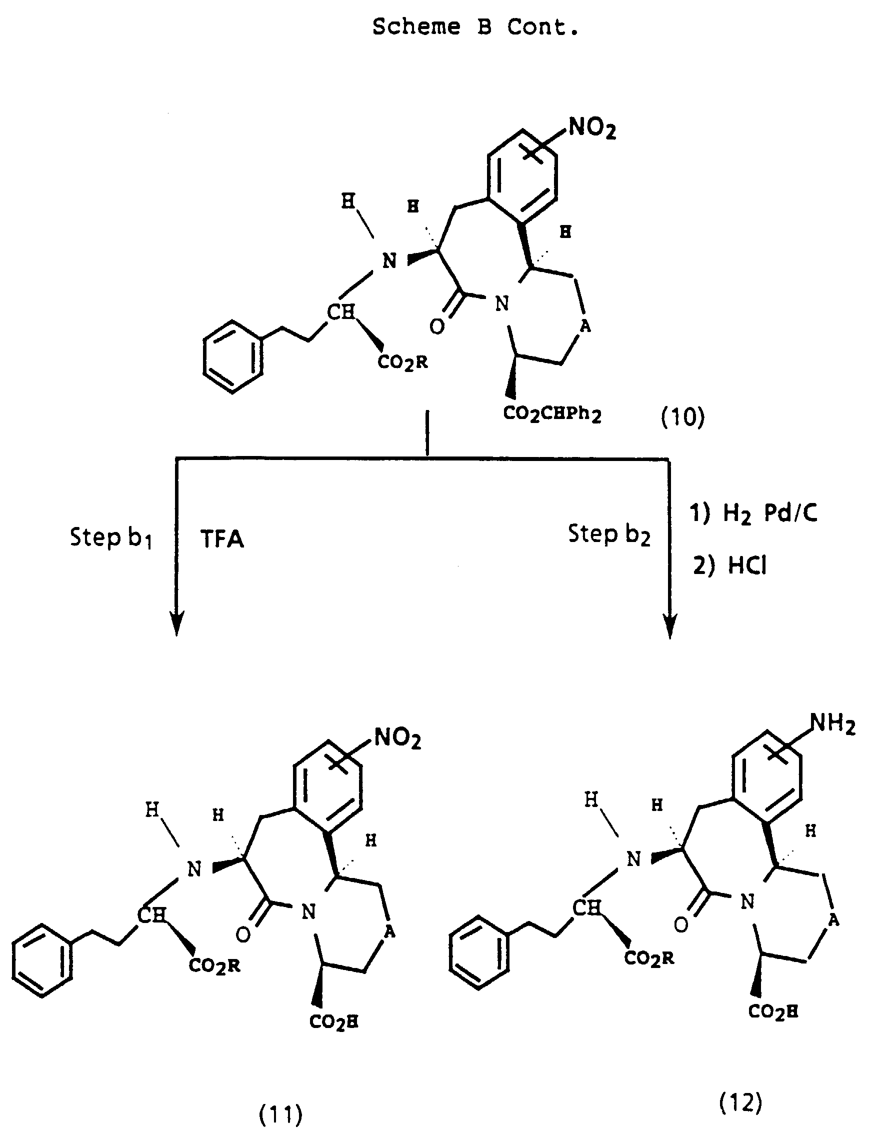

Scheme B provides a general synthetic scheme for preparing compounds of formula (1) wherein R is C₁-C₄ alkyl.

In step a, the amino functionality of the appropriate individual 9-nitro and 11-nitro/amino/carboxylic acid, diphenylmethyl ester compound of structure (4) is alkylated with (R)-2-trifluoromethanesulfonyl-4-phenylbutyric acid, C₁-C₄ alkyl ester (9) to give the corresponding individual 9-nitro and 11-nitro/[1-(carboalkoxy)-3-phenyl]propylamino/carboxylic acid, diphenylmethyl ester compound of structure (10) as described previously in Scheme A, step d.

In step b₁, the carboxylic acid, diphenylmethyl ester functionality of the appropriate individual 9-nitro and 11-nitro/[1-(carboalkoxy)-3-phenyl]propylamino/carboxylic acid, diphenylmethyl ester compound of structure (10) is hydrolyzed to give the corresponding individual 9-nitro and 11-nitro/[1-(carboalkoxy)-3-phenyl]propylamino/carboxylic acid compound of structure (11) as described previously in Scheme A, step e₁.

In step b₂, the nitro functionality of the appropriate individual 9-nitro and 11-nitro/[1-(carboalkoxy)-3-phenyl]propylamino/carboxylic acid, diphenylmethyl ester compound of structure (10) is reduced concurrently with diphenylmethyl ester hydrogenolysis to give the corresponding individual 9-amino and 11-amino/[1-(carboalkoxy)-3-phenyl]propylamino/carboxylic acid compound of structure (12) as described previously in Scheme A, step e₂.

For those compounds of formula (1) wherein A is N-COR₂, the N-COR₂ group of the appropriate individual 9-nitro and 11-nitro/[1-(carboalkoxy)-3-phenyl]propylamino/carboxylic acid compound of structure (11) or the appropriate 9-amino and 11-amino/[1-(carboalkoxy)-3-phenyl]propylamino/carboxylic acid compound of structure (12) may be cleaved by techniques and procedures well known and appreciated in the art, such as lithium hydroxide, to give the corresponding compounds of formula (1) wherein A is NH.

Phthalimide protected amino/carboxylic acid compounds of structure (l) wherein A is O may be prepared as described in Scheme C. In Scheme C, all substituents unless otherwise indicated are as previously defined.

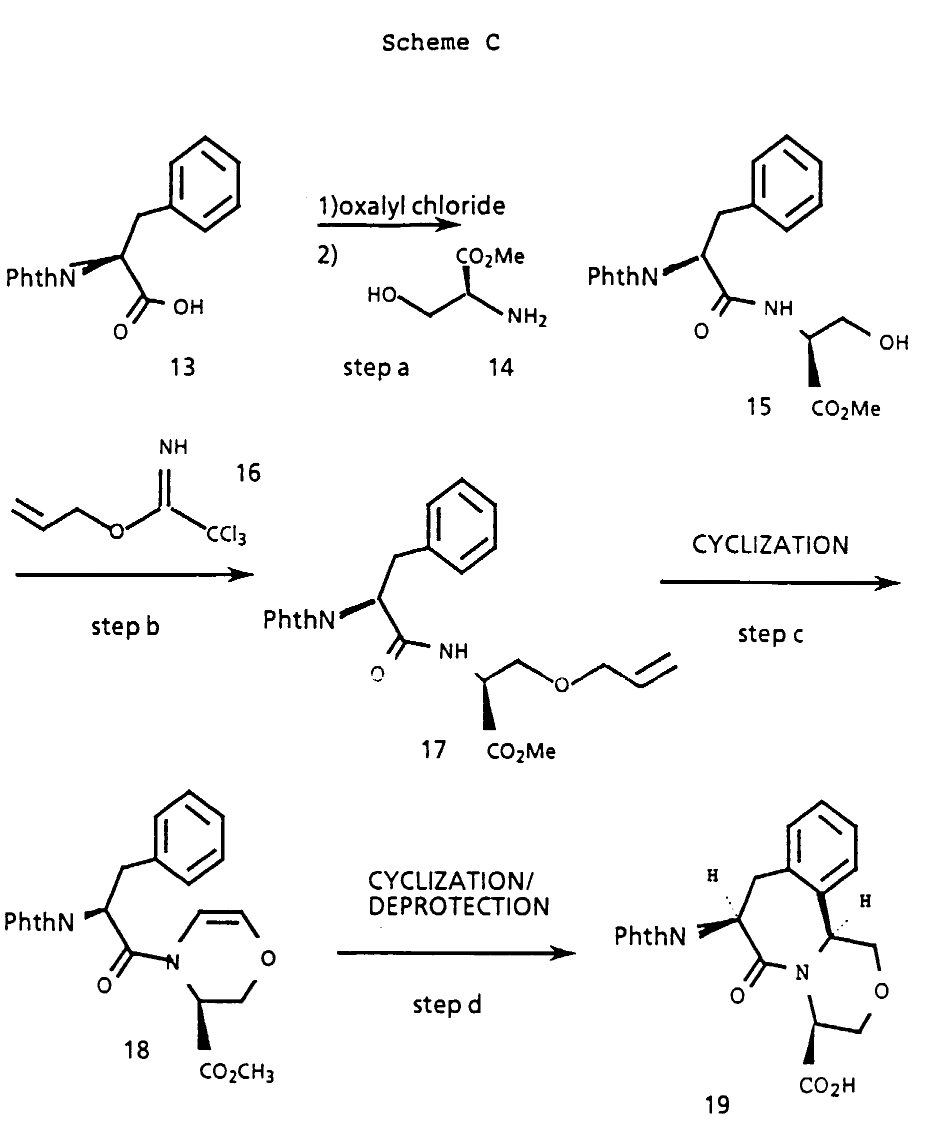

Scheme C provides a general synthetic procedure for preparing phthalimide protected amino/carboxylic acid compounds of structure (l) wherein A is O.

In step a, the appropriate phthalimide blocked (S)phenylalanine derivative of structure (13) is converted to the corresponding acid chloride, then reacted with the appropriate L-serine methyl ester of structure (14) to give the corresponding 1-oxo-3-phenylpropyl-L-serine methyl ester of structure (15).

For example, the appropriate phthalimide blocked (S)phenylalanine derivative of structure (13) can be reacted with oxalyl chloride in a suitable aprotic solvent, such as methylene chloride. The resulting acid chloride can then be coupled with the appropriate L-serine methyl ester of structure (14) using N-methylmorpholine in a suitable aprotic solvent, such as dimethylformamide, to give the appropriate 1-oxo-3-phenylpropyl-L-serine methyl ester of structure (15).

In step b, the hydroxy functionality of the appropriate 1-oxo-3-phenylpropyl-L-serine methyl ester of structure (15) is allylated with the allyl imidate of structure (16) to give the corresponding 1-oxo-3-phenylpropyl-L-serine-O-allyl methyl ester of structure (17).

For example, the appropriate 1-oxo-3-phenylpropyl-L-serine methyl ester of structure (15) is contacted with 2 molar equivalents of the allyl imidate of structure (16) and a molar equivalent of a suitable acid such as trifluoromethanesulfonic acid. The reactants are typically contacted in a suitable organic solvent mixture such as methylene chloride/cyclohexane. The reactants are typically stirred together at room temperature under an inert atmosphere for a period of time ranging from 2-24 hours. The 1-oxo-3-phenylpropyl-L-serine-O-allyl methyl ester of structure (17) is recovered from the reaction zone by extractive methods as is known in the art. It may be purified by silica gel chromatography or crystallization.

In step c, the appropriate 1-oxo-3-phenylpropyl-L-serine-O-allyl methyl ester of structure (17) is cyclized to give the corresponding (4S)-enamine of structure (18).

For example, the appropriate 1-oxo-3-phenylpropyl-L-serine-O-allyl methyl ester of structure (17) is first contacted with a molar excess of a mixture of ozone/oxygen. The reactants are typically contacted in a suitable organic solvent mixture such as methylene chloride/methanol. The reactants are typically stirred together for a period of time ranging from 5 minutes to 30 minutes or until a blue color persists and at a temperature range of from -78°C to -40°C. The reaction is quenched with an excess of methylsulfide and the intermediate aldehyde compound recovered from the reaction zone by extractive methods as is known in the art.

The intermediate aldehyde compound is then contacted with trifluoroacetic acid in a suitable aprotic solvent, such as methylene chloride to give the corresponding (4S)-enamine of structure (18).

In step d, the appropriate (4S)-enamine of structure (18) is cyclized and the methyl ester functionality is removed to give the corresponding phthalimide protected amino/carboxylic acid compound of structure (19) by an acid catalyzed Friedel-Crafts reaction. For example, the appropriate (4S)-enamine of structure (18) can be converted to the corresponding phthalimide protected amino/carboxylic acid compound of structure (19) by treatment with a mixture of trifluoromethane sulfonic acid and trifluoroacetic anhydride in a suitable aprotic solvent, such as methylene chloride.

Phthalimide protected amino/carboxylic acid compounds of structure (l) wherein A is N-B may be prepared as described in Scheme D. In Scheme D, all substituents unless otherwise indicated are as previously defined.

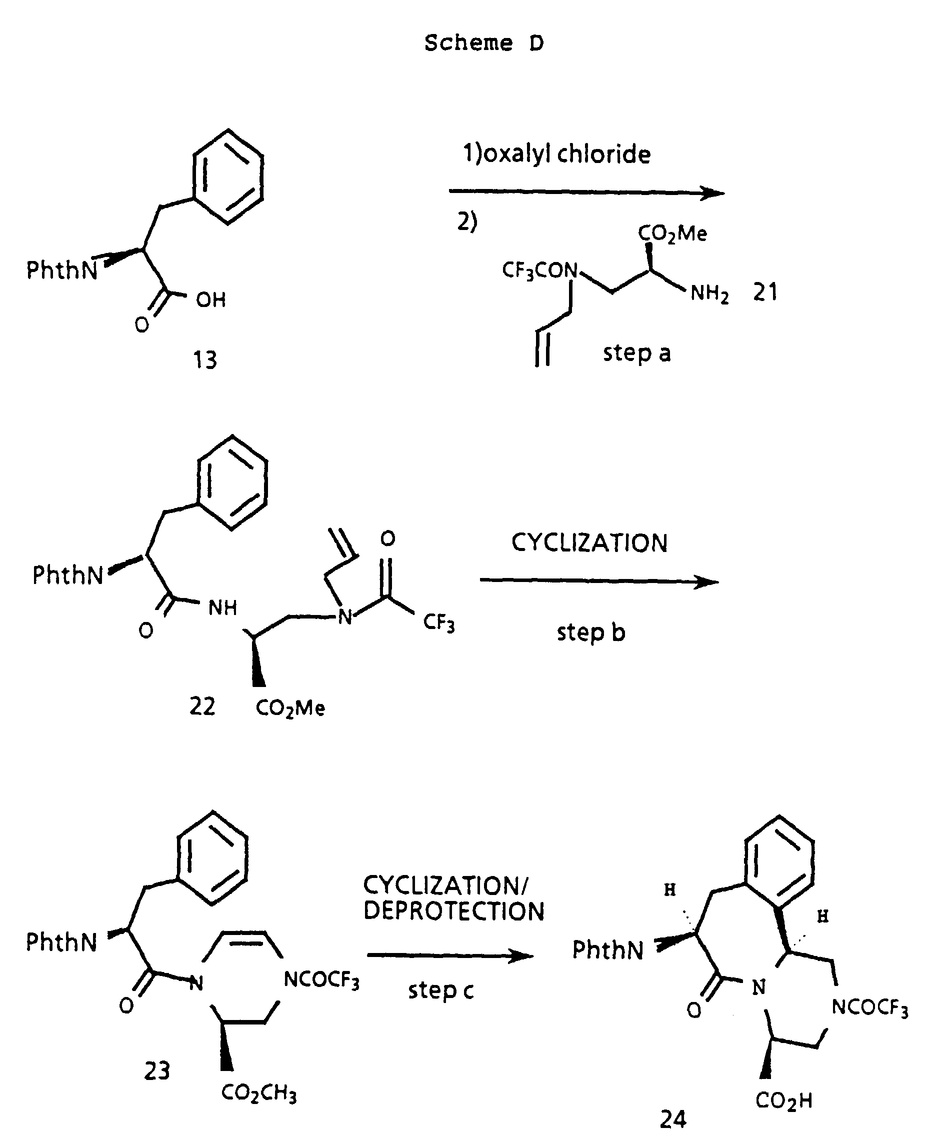

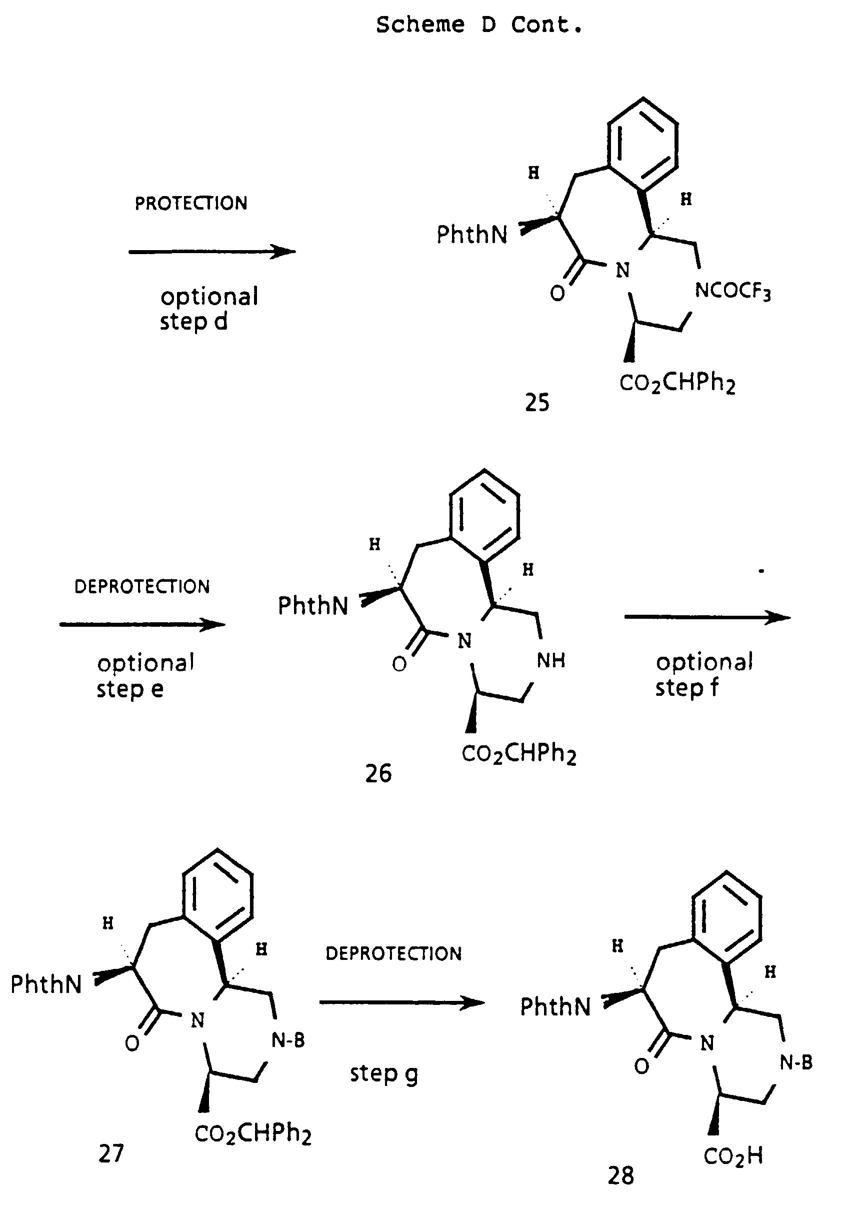

Scheme D provides an alternative general synthetic procedure for preparing phthalimide protected amino/carboxylic acid compounds of structure (l) wherein A is N-B.

In step a, the appropriate phthalimide blocked (S)-phenylalanine derivative of structure (13) is converted to the corresponding acid chloride, then reacted with the appropriate 3-trifluoracetylamino-3-allyl-L-2-aminopropionic acid, methyl ester of structure (21) to give the corresponding 1-oxo-3-phenylpropyl-N-trifluoracetyl-N-allyl-L-amino acid, methyl ester of structure (22) as described previously in Scheme C, step a.

In step b, the appropriate 1-oxo-3-phenylpropyl-N-trifluoracetyl-N-allyl-L-amino acid, methyl ester of structure (22) is cyclized to give the corresponding enamine of structure (23) as described previously in Scheme C, step c.

In step c, the appropriate (4S)-enamine of structure (23) is cyclized to give the corresponding phthalimide protected amino/carboxylic acid compound of structure (24) as described previously in Scheme C, step d.

In optional step d, it is necessary to reesterify the carboxy functionality of the phthalimide protected amino/carboxylic acid compound of structure (24) in order to carry out optional steps e and f. For example, treatment of the crude phthalimide protected amino/carboxylic acid compound of structure (24) with bromodiphenylmethane in a suitable aprotic solvent, such as dimethylformamide along with a non-nucleophilic base, such as cesium carbonate, may be used to give the corresponding phthalimide protected amino/carboxylic acid, diphenylmethyl ester compound of structure (25).

In optional step e, the trifluoroacetate protecting group of the appropriate phthalimide protected amino/carboxylic acid, diphenylmethyl ester compound of structure (25) is removed with a base such as lithium hydroxide as is known in the art to give the corresponding phthalimide protected amino/carboxylic acid, diphenylmethyl ester compound of structure (26).

In optional step f, the free amino functionality of the phthalimide protected amino/carboxylic acid, diphenylmethyl ester compound of structure (26) is alkylated or acylated by techniques and procedures known in the art to give the corresponding phthalimide protected amino/carboxylic acid, diphenylmethyl ester compound of structure (27) wherein B is R₁ or COR₂ which may be used in Scheme A.

In step g, the diphenylmethyl ester functionality of the appropriate phthalimide protected amino/carboxylic acid, diphenylmethyl ester compound of structure (25), the appropriate phthalimide protected amino/carboxylic acid, diphenylmethyl ester compound of structure (26) or the appropriate phthalimide protected amino/carboxylic acid, diphenylmethyl ester compound of structure (27) is removed to give the corresponding phthalimide protected amino/carboxylic acid compound of structure (28) as described previously in Scheme A, step e₁.

Starting materials for use in Schemes A through D are readily available to one of ordinary skill in the art. For example, certain tricyclic amino compounds of structure (l) wherein X is S are described in European Patent 0 249 223 (December 16, 1987) and certain other tricyclic amino compounds of structure (l) wherein A is methylene may be prepared as described in European Patent Application of Flynn and Beight, Application *#34533A EP* (June 11, 1987). Diphenyldiazomethane is described in *Org. Syn.,* Coll. Vol. (III), 351. Certain (R)-2-hydroxy-4-phenylbutyric acid esters are described in U.S. Patent #4,837,354 of Flynn and Beight (June 6, 1989). Ethyl (R)-2-trifluoromethanesulfonyl-4-phenylbutyrate is described in *Tetrahedron Lett.* 25, 1143-46 **1984**.

The following examples present typical syntheses as described in Schemes A through D. These examples are understood to be illustrative only and are not intended to limit the scope of the present invention in any way. As used herein, the following terms have the indicated meanings: "g" refers to grams; "mmol" refers to millimoles; "mL" refers to milliliters; "bp" refers to boiling point; "°C" refers to degrees Celsius; "mm Hg" refers to millimeters of mercury; "µL" refers to microliters; "µg" refers to micrograms; and "µM" refers to micromolar.

### Example 1

### [4S-[4α,7α(R*),12bβ]]-7-[(S-1-Carboxy-3-phenylpropyl)amino]-1,2,3,4,6,7,8,12b-octahydro-9-nitro-6-oxopyrido[2,1-a][2]benzazepine-4-carboxylic acid

### Scheme A, Step a: [4S-[4α,7α(R*),12bβ]]-7-[1,3-Dihydro-1,3-dioxo-2H-isoindol-2-yl]-1,2,3,4,6,7,8,12b-octahydro-9-nitro-6-oxopyrido[2,1-a][2]benzazepine-4-carboxylic acid and [4S-[4α,7α(R*),12bβ]]-7-[1,3-dihydro-1,3-dioxo-2H-isoindol-2 -yl]-1,2,3,4,6,7,8,12b-octahydro-11-nitro-6-oxopyrido[2,1 -a][2]benzazepine-4-carboxylic acid

Dissolve [4S-[4α,7α(R*),12bβ]]-7-[1,3-dihydro-1,3-dioxo-2H-isoindol-2-yl]-1,2,3,4,6,7,8,12b-octahydro-6-oxopyrido[2,1-a][2]benzazepine-4-carboxylic acid (1.46g, 3.5mmol) in methylene chloride (10mL) and cool to -60°C. Add, by dropwise addition, a solution of nitronium tetrafluoroborate (25mL of a 0.5M solution in sulfolane, 12.5mmol) in methylene chloride (15mL). Warm slowly to 10°C over 34 hours. Partition between methylene chloride (75mL) and water (75mL). Separate the aqueous phase and extract with methylene chloride (50mL). Combine the organic phases, dry (MgSO₄) and evaporate the solvent *in vacuo.* Purify and separate by flash silica gel chromatography (1:1 ethyl acetate/hexane ⇒ 1:1 ethyl acetate/hexane with 5% acetic acid ⇒ 2:1 ethyl acetate/hexane with 5% acetic acid) to give the 11-nitro title compound (1.01g, 64.3%) and the 9-nitro title compound (0.43g, 27.4%) as brown foams.

### Scheme A, Step b: [4S-[4α,7α(R*),12bβ]]-7-[1,3-Dihydro-1,3-dioxo-2H-isoindol-2-yl]-1,2,3,4,6,7,8,12b-octahydro-9-nitro-6-oxopyrido[2,1-a][2]benzazepine-4-carboxylic acid, diphenylmethyl ester

Dissolve [4S-[4α,7α(R*),12bβ]]-7-[1,3-dihydro-1,3-dioxo-2H-isoindol-2-yl]-1,2,3,4,6,7,8,12b-octahydro-9-nitro-6-oxopyrido[2,1-a][2]benzazepine-4-carboxylic acid (674mg, 1.50mmol) in methylene chloride (lmL). Add diphenyldiazomethane (291mg, 1.50mmol) and let stand for several days in which time the methylene chloride evaporates. Purify the residue by silica gel chromatography (40% ethyl acetate/hexane ⇒ 55% ethyl acetate/hexane) to give the title compound as a white foam (613mg, 66%).
Anal. Calcd for C₃₆H₂₉N₃O₇: C, 70.23; H, 4.75; N, 6.83;
Found: C, 70.23; H, 4.77; N, 6.63.

### Scheme A, Step c: [4S-[4α,7α(R*),12bβ]]-7-Amino-1,2,3,4,6,7,8,12b-octahydro-9-nitro-6-oxopyrido[2,1-a][2]benzazepine-4-carboxylic acid, diphenylmethyl ester

Slurry [4S-[4α,7α(R*),12bβ]]-7-[1,3-dihydro-1,3-dioxo-2H-isoindol-2-yl]-1,2,3,4,6,7,8,12b-octahydro-9-nitro-6-oxopyrido[2,1-a][2]benzazepine-4-carboxylic acid, diphenylmethyl ester (200mg, 0.324mmol) in methanol (6mL) and add hydrazine hydrate (1.0mL of a 1M solution in methanol, 1.0mmol). Stir at room temperature under an argon atmosphere for 20 hours. Dilute with methylene chloride (30mL) and stir for 2 hours. Filter and concentrate *in vacuo*. Take up the residue in methylene chloride and filter again. Wash the filtrate with water, dry (MgSO₄) and evaporate the solvent *in vacuo* to give the title compound as a yellow foam (165mg).

### Scheme A, Step d: [4S-[4α,7α(R*),12bβ]]-7-[[S-1-[Carbodiphenylmethoxy]-3-phenylpropyl]amino]-1,2,3,4,6,7,8,12b-octahydro-9-nitro-6-oxopyrido[2,1-a][2]benzazepine-4-carboxylic acid, diphenylmethyl ester

Dissolve (R)-2-hydroxy-4-phenylbutyric acid, ethyl ester (4.16g, 20mmol) in methanol (40mL). Add 1N lithium hydroxide (25mL, 25mmol) and stir under an atmosphere of argon for 3.5 hours. Acidify with 1N hydrochloric acid (25mL), saturate with sodium chloride and extract with ethyl acetate (3X25mL). Combine the organic phases, dry (MgSO₄) and evaporate the solvent *in vacuo*. Take up the residue in methylene chloride (20mL) and add diphenyldiazomethane (3.36g, 17.3mmol). Stir overnight and wash with saturated sodium hydrogen carbonate (2X). Dry (MgSO₄), evaporate the solvent *in vacuo* and recrystallize (methylene chloride/hexane then again with cyclohexane) to give (R)-2-hydroxy-4-phenylbutyric acid, diphenylmethyl ester as white needles (2.3g, 38%); mp 82-84°C.
Anal. Calcd for C₂₃H₂₂O₃: C, 79.74; H, 6.40;
Found: C, 79.76; H, 6.41.

Dissolve (R)-2-hydroxy-4-phenylbutyric acid, diphenylmethyl ester (1.38g, 4.0mmol) in methylene chloride (12mL). Add pyridine (0.32mL, 4mmol), place under an argon atmosphere and cool to -10°C. Add, by dropwise addition, a solution of trifluoromethanesulfonic anhydride (0.673mL, 4.0mmol) in methylene chloride (2mL). Stir at -10 to -20°C for 2 hours, dilute with hexane (10mL) and filter. Evaporate the solvent *in vacuo* to give (R)-2-trifluoromethanesulfonyl-4-phenylbutyric acid, diphenylmethyl ester.

Dissolve [4S-[4α,7α(R*),12bβ]]-7-amino-1,2,3,4,6,7,8,12b-octahydro-9-nitro-6-oxopyrido[2,1-a][2]benzazepine-4-carboxylic acid, diphenylmethyl ester (158mg, 0.632mmol) and proton sponge (138mg, 0.648mmol) in methylene chloride (5mL). Add a solution of (R)-2-trifluoromethanesulfonyl-4-phenylbutyric acid, diphenylmethyl ester (309mg, 0.648mmol) in methylene chloride (2ml). Stir at room temperature under an argon atmosphere for 26 hours. Purify by flash silica gel chromatography (30% ethyl acetate/hexane ⇒ 50% ethyl acetate/hexane) to give the title compound as a yellow foam (178mg, 67.2%).
Anal. Calcd for C₅₁H₄₇N₃O₇: C, 75.25; H, 5.82; N, 5.16;
Found: C, 74.10; H, 5.82; N, 4.87.

### Scheme A, Step e₁: [4S-[4α,7α(R*),12bβ]]-7-[(S-1-Carboxy-3-phenylpropyl)amino]-1,2,3,4,6,7,8,12b-octahydro-9-nitro-6-oxopyrido[2,1-a][2]benzazepine-4-carboxylic acid

Dissolve [4S-[4α,7α(R*),12bβ]]-7-[[S-1-[carbodiphenylmethoxy]-3-phenylpropyl]amino]-1,2,3,4,6,7,8,12b-octahydro-9-nitro-6-oxopyrido[2,1-a][2]benzazepine-4-carboxylic acid, diphenylmethyl ester (39mg, 0.045mmol) in methylene chloride (1mL). Add trifluoroacetic acid (lmL) and anisole (0.2mL). Allow to stand for 3 hours under an argon atmosphere. Partition between methylene chloride and 1N hydrochloric acid. Separate the aqueous phase and freeze dry. Purify by High Pressure Liquid Chromatography (elute with 25% methanol with 0.1% trifluoroacetic acid at 15mL/min on a 22.5cm X250cm Vydac C-18 column) to give the title compound (18mg, 72%).

### Example 2

### [4S-[4α,7α(R*),12bβ]]-9-Amino-7-[(S-1-carboxy-3-phenylpropyl)amino]-1,2,3,4,6,7,8,12b-octahydro-6-oxopyrido[2,1-a][2]benzazepine-4-carboxylic acid, dihydrochloride

Dissolve [4S-[4α,7α(R*),12bβ]]-7-[[S-1-[carbodiphenylmethoxy]-3-phenylpropyl]amino]-1,2,3,4,6,7,8,12b-octahydro-9-nitro-6-oxopyrido[2,1-a][2]benzazepine-4-carboxylic acid, diphenylmethyl ester (52mg, 0.064mmol) in tetrahydrofuran (3mL). Add 10% palladium/carbon (18mg) and water (3mL). Shake under 44 psi of hydrogen at room temperature for 20 hours. Filter and evaporate the solvent *in vacuo*. Take the residue up in 1N hydrochloric acid, filter then freeze dry to give the title compound (25mg, 74%).

### Example 3

### [4S-[4α,7α(R*),12bβ]]-7-[(S-1-Carboxy-3-phenylpropyl)amino]-1,2,3,4,6,7,8,12b-octahydro-11-nitro-6-oxopyrido[2,1-a][2]benzazepine-4-carboxylic acid

### Scheme A, Step b: [4S-[4α,7α(R*),12bβ]]-7-[1,3-Dihydro-1,3-dioxo-2H-isoindol-2-yl]-1,2,3,4,6,7,8,12b-octahydro-11-nitro-6-oxopyrido[2,1-a][2]benzazepine-4-carboxylic acid, diphenylmethyl ester

Dissolve [4S-[4α,7α(R*),12bβ]]-7-[1,3-dihydro-1,3-dioxo-2H-isoindol-2-yl]-1,2,3,4,6,7,8,12b-octahydro-11-nitro-6-oxopyrido[2,1-a][2]benzazepine-4-carboxylic acid (795mg, 1.77mmol) in methylene chloride (2mL). Add diphenyldiazomethane (350mg, 1.78mmol) and methylene chloride (5mL). Let stand for 8 hours. Purify the residue by silica gel chromatography (35% ethyl acetate/hexane ⇒ 50% ethyl acetate/hexane) to give the title compound as a white foam (756mg, 69.4%).

### Scheme A, Step c: [4S-[4α,7α(R*),12bβ]]-7-Amino-1,2,3,4,6,7,8,12b-octahydro-11-nitro-6-oxopyrido[2,1-a][2]benzazepine-4-carboxylic acid, diphenylmethyl ester

Slurry [4S-[4α,7α(R*),12bβ]]-7-[1,3-dihydro-1,3-dioxo-2H-isoindol-2-yl]-1,2,3,4,6,7,8,12b-octahydro-11-nitro-6-oxopyrido[2,1-a][2]benzazepine-4-carboxylic acid, diphenylmethyl ester (756mg, 1.23mmol) in methanol (5mL) and add hydrazine hydrate (4.0mL of a 1M solution in methanol, 4.0mmol). Stir at room temperature under an argon atmosphere for 10 minutes then warm to reflux for 90 minutes. Evaporate the solvent *in vacuo* and stir the residue with methylene chloride for 20 minutes. Filter and wash the filtrate with water, dry (MgSO₄) and evaporate the solvent *in vacuo* to give the title compound as a yellow film (610mg, 100%).

### Scheme A, Step d: [4S-[4α,7α(R*),12bβ]]-7-[[S-1-[Carbodiphenylmethoxy]-3-phenylpropyl]amino]-1,2,3,4,6,7,8,12b-octahydro-11-nitro-6-oxopyrido[2,1-a][2]benzazepine-4-carboxylic acid, diphenylmethyl ester

Dissolve [4S-[4α,7α(R*),12bβ]]-7-amino-1,2,3,4,6,7,8,12b-octahydro-11-nitro-6-oxopyrido[2,1-a][2]benzazepine-4-carboxylic acid, diphenylmethyl ester (90mg, 0.18mmol) and proton sponge (250mg, 1.12mmol) in methylene chloride (5mL). Add a solution of (R)-2-trifluoromethanesulfonyl-4-phenylbutyric acid, diphenylmethyl ester (520mg, 1.17mmol) in methylene chloride (6ml). Stir at room temperature under an argon atmosphere for 22 hours. Dilute with cyclohexane (20mL) and filter. Purify by silica gel chromatography (30% ethyl acetate/hexane) to give the title compound (105mg, 72%).
Anal. Calcd for C₅₁H₄₇N₃O₇: C, 75.25; H, 5.82; N, 5.16;
Found: C, 74.84; H, 5.84; N, 4.84.

### Scheme A, Step e₁: [4S-[4α,7α(R*),12bβ]]-7-[(S-1-Carboxy-3-phenylpropyl)amino]-1,2,3,4,6,7,8,12b-octahydro-11-nitro-6-oxopyrido[2,1-a][2]benzazepine-4-carboxylic acid, hydrochloride

Dissolve [4S-[4α,7α(R*),12bβ]]-7-[[S-1-[carbodiphenylmethoxy]-3-phenylpropyl]amino]-1,2,3,4,6,7,8,12b-octahydro-11-nitro-6-oxopyrido[2,1-a][2]benzazepine-4-carboxylic acid, diphenylmethyl ester (52mg, 0.063mmol) in methylene chloride (2mL). Add trifluoroacetic acid (1mL) and anisole (0.2mL). Allow to stand for 2 hours under an argon atmosphere. Partition between methylene chloride and 9:1 water/methanol. Separate the organic phase and extract with additional 9:1 water/methanol (10mL). Combine the aqueous phases and wash with methylene chloride (3X10mL). Separate the aqueous phase and freeze dry to give the title compound as a white powder (24.6mg, 74.5%).

### Example 4

### [4S-[4α,7α(R*),12bβ]]-11-Amino-7-[(S-1-carboxy-3-phenylpropyl)amino]-1,2,3,4,6,7,8,12b-octahydro-6-oxopyrido[2,1-a][2]benzazepine-4-carboxylic acid, dihydrochloride

Dissolve [4S-[4α,7α(R*),12bβ]]-7-[[S-1-[carbodiphenylmethoxy]-3-phenylpropyl]amino]-1,2,3,4,6,7,8,12b-octahydro-11-nitro-6-oxopyrido[2,1-a][2]benzazepine-4-carboxylic acid, diphenylmethyl ester (56mg, 0.069mmol) in tetrahydrofuran (6mL). Add 10% palladium/carbon (20mg) and water (3mL). Shake under 45 psi of hydrogen at room temperature for 5 hours. Filter and partition the filtrate between 1N hydrochloric acid and methylene chloride. Separate the aqueous phase and freeze dry to give the title compound (35.3mg, 98%).

### Example 5

### [4S-[4α,7α(R*),12bβ]]-7-[(S-1-Carbethoxy-3-phenylpropyl)amino]-1,2,3,4,6,7,8,12b-octahydro-9-nitro-6-oxopyrido[2,1-a][2]benzazepine-4-carboxylic acid

### Scheme B, Step a: [4S-[4α,7α(R*),12bβ]]-7-[(S-1-Carbethoxy-3-phenylpropyl)amino]-1,2,3,4,6,7,8,12b-octahydro-9-nitro-6-oxopyrido[2,1-a][2]benzazepine-4-carboxylic acid, diphenylmethyl ester

Dissolve [4S-[4α,7α(R*),12bβ]]-7-amino-1,2,3,4,6,7,8,12b-octahydro-9-nitro-6-oxopyrido[2,1-a][2]benzazepine-4-carboxylic acid, diphenylmethyl ester (596mg, 1.23mmol) and proton sponge (296mg, 1.25mmol) in methylene chloride (10mL). Add a solution of (R)-2-trifluoromethanesulfonyl-4-phenylbutyric acid, ethyl ester (428mg, 1.25mmol) in methylene chloride (6ml). Stir at room temperature under an argon atmosphere overnight. Purify by silica gel chromatography to give the title compound.

### Scheme B, Step b₁: [4S-[4α,7α(R*),12bβ]]-7-[(S-1-Carbethoxy-3-phenylpropyl)amino]-1,2,3,4,6,7,8,12b-octahydro-9-nitro-6-oxopyrido[2,1-a][2]benzazepine-4-carboxylic acid

Dissolve [4S-[4α,7α(R*),12bβ]]-7-[(S-1-carbethoxy-3-phenylpropyl)amino]-1,2,3,4,6,7,8,12b-octahydro-9-nitro-6-oxopyrido[2,1-a][2]benzazepine-4-carboxylic acid, diphenylmethyl ester (42.5mg, 0.063mmol) in methylene chloride (2mL). Add trifluoroacetic acid (1mL) and anisole (0.2mL). Allow to stand for 2 hours under an argon atmosphere. Partition between methylene chloride and 9:1 water/methanol. Separate the organic phase and extract with additional 9:1 water/methanol (10mL). Combine the aqueous phases and wash with methylene chloride (3X10mL). Separate the aqueous phase and freeze dry to give the title compound.

### Example 6

### [4S-[4α,7α(R*),12bβ]]-9-Amino-7-[(1-carbethoxy-3-phenylpropyl)amino]-1,2,3,4,6,7,8,12b-octahydro-6-oxopyrido[2,1-a][2]benzazepine-4-carboxylic acid dihydrochloride

Dissolve [4S-[4α,7α(R*),12bβ]]-7-[(S-1-carbethoxy-3-phenylpropyl)amino]-1,2,3,4,6,7,8,12b-octahydro-9-nitro-6-oxopyrido[2,1-a][2]benzazepine-4-carboxylic acid, diphenylmethyl ester (260mg, 385mmol) in ethanol (7mL). Add 10% palladium/carbon under an atmosphere of carbon dioxide. Shake under 42 psi of hydrogen at room temperature for 5.5 hours. Filter and evaporate the solvent *in vacuo.* Take up the residue in 1N hydrochloric acid (2mL) and dilute with water (10mL). Wash with methylene chloride (3X5mL) and freeze dry the aqueous phase to give the title compound.

### Example 7

### [4S-[4α,7α(R*),12bβ]]-7-[(S-1-Carbethoxy-3-phenylpropyl)amino]-1,2,3,4,6,7,8,12b-octahydro-11-nitro-6-oxopyrido[2,1-a][2]benzazepine-4-carboxylic acid

### Scheme B, Step a: [4S-[4α,7α(R*),12bβ]]-7-[(S-1-Carbethoxy-3-phenylpropyl)amino]-1,2,3,4,6,7,8,12b-octahydro-11-nitro-6-oxopyrido[2,1-a][2]benzazepine-4-carboxylic acid, diphenylmethyl ester

Dissolve [4S-[4α,7α(R*),12bβ]]-7-amino-1,2,3,4,6,7,8,12b-octahydro-11-nitro-6-oxopyrido[2,1-a][2]benzazepine-4-carboxylic acid, diphenylmethyl ester (596mg, 1.23mmol) and proton sponge (296mg, 1.25mmol) in methylene chloride (10mL). Add a solution of (R)-2-trifluoromethanesulfonyl-4-phenylbutyric acid, ethyl ester (428mg, 1.25mmol) in methylene chloride (6ml). Stir at room temperature under an argon atmosphere overnight. Purify by silica gel chromatography (35% ethyl acetate/hexane) to give the title compound as a light yellow foam (591mg, 71.1%); mp 75-80°C.
Anal. Calcd for C₄₀H₄₁N₃O₇: C, 71.09; H, 6.12; N, 6.22;
Found: C, 70.81; H, 5.98; N, 6.13.

### Scheme B, Step b₁: [4S-[4α,7α(R*),12bβ]]-7-[(S-1-Carbethoxy-3-phenylpropyl)amino]-1,2,3,4,6,7,8,12b-octahydro-11-nitro-6-oxopyrido[2,1-a][2]benzazepine-4-carboxylic acid

Dissolve [4S-[4α,7α(R*),12bβ]]-7-[(S-1-carbethoxy-3-phenylpropyl)amino]-1,2,3,4,6,7,8,12b-octahydro-11-nitro-6-oxopyrido[2,1-a][2]benzazepine-4-carboxylic acid, diphenylmethyl ester (42.5mg, 0.063mmol) in methylene chloride (2mL). Add trifluoroacetic acid (lmL) and anisole (0.2mL). Allow to stand for 2 hours under an argon atmosphere. Partition between methylene chloride and 9:1 water/methanol. Separate the organic phase and extract with additional 9:1 water/methanol (10mL). Combine the aqueous phases and wash with methylene chloride (3X10mL). Separate the aqueous phase and freeze dry to give the title compound.

### Example 8

### [4S-[4α,7α(R*),12bβ]]-11-Amino-7-[(S-1-carbethoxy-3-phenylpropyl)amino]-1,2,3,4,6,7,8,12b-octahydro-6-oxopyrido[2,1-a][2]benzazepine-4-carboxylic acid, dihydrochloride

Dissolve [4S-[4α,7α(R*),12bβ]]-7-[(S-1-carbethoxy-3-phenylpropyl)amino]-1,2,3,4,6,7,8,12b-octahydro-11-nitro-6-oxopyrido[2,1-a][2]benzazepine-4-carboxylic acid diphenylmethyl ester (260mg, 385mmol) in ethanol (7mL). Add 10% palladium/carbon under an atmosphere of carbon dioxide. Shake under 42 psi of hydrogen at room temperature for 5.5 hours. Filter and evaporate the solvent *in vacuo*. Take up the residue in 1N hydrochloric acid (2mL) and dilute with water (10mL). Wash with methylene chloride (3X5mL) and freeze dry the aqueous phase to give the title compound as yellow crystals (178mg, 83.6%); mp 175-95°C.

### Example 9

### [4S-[4α,7α(R*),12bβ]]-7-[(S-1-Carboxy-3-phenylpropyl)amino]-1,2,3,4,6,7,8,12b-hexahydro-9-nitro-6-oxo-1H-[1,4]-oxazino[3,4-a][2]benzazepine-4-carboxylic acid

### Scheme C, step a: N-[2-(1,3-Dihydro-1,3-dioxo-2H-isoindol-2-yl)-1-oxo-3-phenylpropyl]-L-serine, methyl ester

Slurry N-phthaloyl-(S)-phenylalanine (90g, 0.3mol) in methylene chloride (450mL) and add, by dropwise addition, oxalyl chloride (54mL, 0.62mol). Place under a dry atmosphere (CaSO₄ tube) and treat with dimethylformamide (10µL). Stir for 5 hours, filter and concentrate in vacuo to give N-phthaloyl-(S)-phenylalanine, acid chloride as an off white amorphous solid.

Dissolve serine methyl ester hydrochloride (56g, 0.36mol) in tetrahydrofuran (300mL) then cool to 0°C and add 4-methylmorpholine (88mL, 0.8mol). Add, by dropwise addition, a solution of the N-phthaloyl-(S)-phenylalanine, acid chloride in tetrahydrofuran (200mL). Allow to warm to room temperature and stir for 3 hours. Filter and concentrate the filtrate in vacuo. Dissolve the residue in ethyl acetate and separate the organic phase. Wash with water then saturated sodium chloride and dry (MgSO₄). Evaporate the solvent in vacuo to give an oil. Purify by silica gel chromatography (gradient 50% ethyl acetate/hexane to ethyl acetate) to give the title compound (80.8g, 67%) mp 129-132°C.

### Scheme C, step b: N-[2-(1,3-Dihydro-1,3-dioxo-2H-isoindol-2-yl)-1-oxo-3-phenylpropyl]-O-2-propenyl-L-serine, methyl ester

Dissolve N-[2-(1,3-dihydro-1,3-dioxo-2H-isoindol-2-yl)-1-oxo-3-phenylpropyl]-L-serine, methyl ester (25g, 63mmol) in methylene chloride/cyclohexane (1:1, 600mL). Add allyl trichloroacetimidate (26g, 128mmol) and trifluoromethanesulfonic acid (5mL, 56.6mmol). Stir at room temperature under a nitrogen atmosphere for 5 hours and dilute with methylene chloride. Wash with saturated aqueous sodium hydrogen carbonate, water, dry (MgSO₄) and evaporate the solvent in vacuo. Purify by silica gel chromatography (gradient 20% ethyl acetate/hexane to 35% ethyl acetate/hexane) to give the title compound; mp 95-97°C.

### Scheme C, step c: [S-(R*, R*)]-N-[2-(1,3-Dihydro-1,3-dioxo-2H-isoindol-2-yl)-1-oxo-3-phenylpropyl]-3,4-dihydro-2H-1,4-oxazine-3-carboxylic acid, methyl ester

Dissolve N-[2-(1,3-dihydro-1,3-dioxo-2H-isoindol-2-yl)-1-oxo-3-phenylpropyl]-O-2-propenyl-L-serine, methyl ester (13g, 29.8mmol) in methylene chloride/methanol (10:1, 220mL). Cool to -78°C and sparge with a mixture of ozone/oxygen for approximately 10 minutes until a blue color persists. Sparge with nitrogen for 10 minutes at -78°C to remove excess ozone. Treat with methyl sulfide (60mL, 0.82mol) and allow to warm to room temperature. Stir at room temperature for 2.5 hours, evaporate the solvent in vacuo and dissolve the residue in ethyl acetate (200mL). Wash with water, saturated sodium chloride, dry (MgSO₄) and evaporate the solvent in vacuo to give the intermediate N-[2-(1,3-dihydro-1,3-dioxo-2H-isoindol-2-yl)-1-oxo-3-phenylpropyl]-O-2-oxoethyl-L-serine, methyl ester as a foam (13.6g).

Dissolve N-[2-(1,3-dihydro-1,3-dioxo-2H-isoindol-2-yl)-1-oxo-3-phenylpropyl]-O-2-oxoethyl-L-serine, methyl ester (13.6g) in methylene chloride/trifluoroacetic acid (10:1/330mL). Stir at room temperature for 2.5 hours and evaporate the solvent in vacuo. Purify by silica gel chromatography (35% ethyl acetate/hexane) and recrystallize (ethyl acetate/hexane) to give the title compound (8.52g, 68%); mp 70-72°C.

### Scheme C, step d: [4S-[4α,7α(R*), 12bβ]]-7-(1,3-Dihydro-1,3-dioxo-2H-isoindol-2-yl)-3,4,6,7,8,12b-hexahydro-6-oxo-1H-[1,4]-oxazino[3,4-a][2]benzazepine-4-carboxylic acid

Dissolve [S-(R*, R*)]-N-[2-(1,3-dihydro-1,3-dioxo-2H-isoindol-2-yl)-1-oxo-3-phenylpropyl]-3,4-dihydro-2H-1,4-oxazine-3-carboxylic acid, methyl ester (2.5g, 5.9mmol) in methylene chloride (5mL) and add, by dropwise addition, to a previously prepared solution of trifluoromethanesulfonic acid (4.0mL, 45mmol) and trifluoroacetic anhydride (1.0mL, 7.1mmol). Place under a nitrogen atmosphere and stir at room temperature for 123 hours. Pour into a separatory funnel containing ice (200g) and ethyl acetate (200mL). Separate the organic phase, wash with water (3X200mL) and saturated aqueous sodium chloride (100mL). Extract the organic phase with 10% wt. potassium hydrogen carbonate (4X40mL) and water (40mL). Layer the combined basic aqueous phases with ethyl acetate (100mL) and cool in an ice bath. Add, by dropwise addition, 6N hydrochloric acid to adjust the pH to 1 while maintaining the temperature at 5-10°C. Separate the organic phase and extract the aqueous phase with ethyl acetate (3X200mL), wash with saturated sodium chloride and dry (MgSO₄). Evaporate the solvent in vacuo and dry the residue under high vacuum at 56°C for 24 hours to give the title compound (1.75g, 73%).

### Scheme A, Step a: [4S-[4α,7α(R*),12bβ]]-7-[1,3-Dihydro-1,3-dioxo-2H-isoindol-2-yl]-3,4,6,7,8,12b-hexahydro-9-nitro-6-oxo-1H-[1,4]-oxazino[3,4-a][2]benzazepine-4-carboxylic acid and [4S-[4α,7α(R*),12bβ]]-7-[1,3-dihydro-1,3-dioxo-2H-isoindol-2-yl]-3,4,6,7,8,12b-hexahydro-11-nitro-6-oxo-1H-[1,4]-oxazino[3,4-a][2]benzazepine-4-carboxylic acid

Mix trifluromethanesulfonic acid (4ml, 45mmol) and nitric acid (lml, 22mmol) and anhydrous methylene chloride (100mL). Cool to -78°C and add, by dropwise addition, a solution of [4S-[4α,7α(R*),12bβ]]-7-[1,3-dihydro-1,3-dioxo-2H-isoindol-2-yl]-3,4,6,7,8,12b-hexahydro-6-oxo-1H-[1,4]-oxazino[3,4-a][2]benzazepine-4-carboxylic acid (1.94g, 4.78mmol) in methylene chloride (60mL). Stir at -78°C for 20 minutes, allow to warm to room temperature and stir under a nitrogen atmosphere for 3.5 hours. Wash with water, dry (MgSO₄) and evaporate the solvent *in vacuo* to give yellow foam (2.3g). Take up in ethyl acetate, wash with brine (3X), dry (MgSO₄) and evaporate the solvent *in vacuo* to give a yellow foam. Purify and separate by flash silica gel chromatography (1:1:0.04 ethyl acetate/hexane/acetic acid) to give a small amount of the 9-nitro title compound and 1.02g of the 11-nitro title compound (47%).

### Scheme A, Step b: [4S-[4α,7α(R*),12bβ]]-7-[1,3-Dihydro-1,3-dioxo-2H-isoindol-2-yl]-3,4,6,7,8,12b-hexahydro-9-nitro-6-oxo-1H-[1,4]-oxazino[3,4-a][2]benzazepine-4-carboxylic acid, diphenylmethyl ester

Dissolve [4S-[4α,7α(R*),12bβ]]-7-[1,3-dihydro-1,3-dioxo-2H-isoindol-2-yl]-3,4,6,7,8,12b-hexahydro-9-nitro-6-oxo-1H-[1,4]-oxazino[3,4-a][2]benzazepine-4-carboxylic acid (1.50mmol) in methylene chloride (1mL). Add diphenyldiazomethane (291mg, 1.50mmol) and let stand for several days in which time the methylene chloride evaporates. Purify the residue by silica gel chromatography to give the title compound.

### Scheme A, Step c: [4S-[4α,7α(R*),12bβ]]-7-Amino-3,4,6,7,8,12b-hexahydro-9-nitro-6-oxo-1H-[1,4]-oxazino[3,4-a][2]benzazepine-4-carboxylic acid, diphenylmethyl ester

Slurry [4S-[4α,7α(R*),12bβ]]-7-[1,3-dihydro-1,3-dioxo-2H-isoindol-2-yl]-3,4,6,7,8,12b-hexahydro-9-nitro-6-oxo-1H-[1,4]-oxazino[3,4-a][2]benzazepine-4-carboxylic acid, diphenylmethyl ester (600mg, 0.97mmol) in methanol (5mL) and add hydrazine hydrate (2.0mL of a 1M solution in methanol, 2.0mmol). Stir at room temperature under an argon atmosphere for 4 hours. Add methanol (20mL) and reflux for 1 hour, then stir at room temperature overnight. Evaporate the solvent *in vacuo*, take up the residue in ethyl acetate and filter. Wash with filtrate with water (3X) and brine. Dry (MgSO₄) and evaporate the solvent *in vacuo* to give the title compound (473mg, 99.5%).

### Scheme A, Step d: [4S-[4α,7α(R*),12bβ]]-7-[[S-1-[Carbodiphenylmethoxy]-3-phenylpropyl]amino]-3,4,6,7,8,12b-hexahydro-9-nitro-6-oxo-1H-[1,4]-oxazino[3,4-a][2]benzazepine-4-carboxylic acid, diphenylmethyl ester

Dissolve [4S-[4α,7α(R*),12bβ]]-7-amino-3,4,6,7,8,12b-hexahydro-9-nitro-6-oxo-1H-[1,4]-oxazino[3,4-a][2]benzazepine-4-carboxylic acid, diphenylmethyl ester (0.632mmol) and proton sponge (138mg, 0.648mmol) in methylene chloride (5mL). Add a solution of (R)-2-trifluoromethanesulfonyl-4-phenylbutyric acid, diphenylmethyl ester (309mg, 0.648mmol) in methylene chloride (2ml). Stir at room temperature under an argon atmosphere for 26 hours. Purify by flash silica gel chromatography to give the title compound.

### Scheme A, Step e₁: [4S-[4α,7α(R*),12bβ]]-7-[(S-1-Carboxy-3-phenylpropyl)amino]-3,4,6,7,8,12b-hexahydro-9-nitro-6-oxo-1H-[1,4]-oxazino[3,4-a][2]benzazepine-4-carboxylic acid

Dissolve [4S-[4α,7α(R*),12bβ]]-7-[[S-1-[(diphenylmethoxy)carbonyl]-3-phenylpropyl]amino]-3,4,6,7,8,12b-hexahydro-9-nitro-6-oxo-1H-[1,4]-oxazino[3,4-a][2]benzazepine-4-carboxylic acid, diphenylmethyl ester (0.045mmol) in methylene chloride (1mL). Add trifluoroacetic acid (lmL) and anisole (0.2mL). Allow to stand for 3 hours under an argon atmosphere. Partition between methylene chloride and 1N hydrochloric acid. Separate the aqueous phase and freeze dry. Purify by High Pressure Liquid Chromatography to give the title compound.

### Example 10

### [4S-[4α,7α(R*),12bβ]]-7-[(S-1-Carboxy-3-phenylpropyl)amino]-3,4,6,7,8,12b-hexahydro-9-nitro-6-oxo-1H-[1,4]-thiazino[3,4-a][2]benzazepine-4-carboxylic acid

### Scheme A, Step a: [4S-[4α,7α(R*),12bβ]]-7-[1,3-Dihydro-1,3-dioxo-2H-isoindol-2-yl]-3,4,6,7,8,12b-hexahydro-9-nitro-6-oxo-1H-[1,4]-thiazino[3,4-a][2]benzazepine-4-carboxylic acid and [4S-[4α,7α(R*),12bβ]]-7-[1,3-dihydro-1,3-dioxo-2H-isoindol-2-yl]-3,4,6,7,8,12b-hexahydro-11-nitro-6-oxo-1H-[1,4]-thiazino[3,4-a][2]benzazepine-4-carboxylic acid

Dissolve [4S-[4α, 7α(R*), 12bβ]]-7-[1,3-dihydro-1,3-dioxo-2H-isoindol-2-yl]-3,4,6,7,8,12b-hexahydro-6-oxo-1H-[1,4]-thiazino[3,4-a][2]benzazepine-4-carboxylic acid, methyl ester (0.233mmol) in ethanol (4mL) and treat with trifluoromethanesulfonic acid (0.5mL). Stir under nitrogen atmosphere until hydrolysis is complete, pour into water, extract the aqueous phases with ethyl acetate (2X) and wash the combined organic phases with water, then with saturated sodium chloride. Dry (MgSO₄), evaporate the solvent *in vacuo* and purify by silica gel chromatography to give [4S-[4α, 7α(R*), 12bβ]]-7-[1,3-dihydro-1,3-dioxo-2H-isoindol-2-yl]-3,4,6,7,8,12b-hexahydro-6-oxo-1H-[1,4]-thiazino[3,4-a][2]benzazepine-4-carboxylic acid.

Mix trifluromethanesulfonic acid (4ml, 45mmol) and nitric acid (1ml, 22mmol) and anhydrous methylene chloride (100mL). Cool to -78°C and add, by dropwise addition, a solution of [4S-[4α,7α(R*),12bβ]]-7-[1,3-dihydro-1,3-dioxo-2H-isoindol-2-yl]-3,4,6,7,8,12b-hexahydro-6-oxo-1H-[1,4]-thiazino[3,4-a][2]benzazepine-4-carboxylic acid (4.78mmol) in methylene chloride (60mL). Stir at -78°C for 20 minutes, allow to warm to room temperature and stir under a nitrogen atmosphere for 3.5 hours. Wash with water, dry (MgSO₄) and evaporate the solvent *in vacuo*. Purify and separate by flash silica gel chromatography to give the 11-nitro title compound and the 9-nitro title compound.

### Scheme A, Step b: [4S-[4α,7α(R*),12bβ]]-7-[1,3-Dihydro-1,3-dioxo-2H-isoindol-2-yl]-3,4,6,7,8,12b-hexahydro-9-nitro-6-oxo-1H-[1,4]-thiazino[3,4-a][2]benzazepine-4-carboxylic acid, diphenylmethyl ester

Dissolve [4S-[4α,7α(R*),12bβ]]-7-[1,3-dihydro-1,3-dioxo-2H-isoindol-2-yl]-3,4,6,7,8,12b-hexahydro-9-nitro-6-oxo-1H-[1,4]-thiazino[3,4-a][2]benzazepine-4-carboxylic acid (1.50mmol) in methylene chloride (lmL). Add diphenyldiazomethane (291mg, 1.50mmol) and let stand for several days in which time the methylene chloride evaporates. Purify the residue by silica gel chromatography to give the title compound.

### Scheme A, Step c: [4S-[4α,7α(R*),12bβ]]-7-Amino-3,4,6,7,8,12b-hexahydro-9-nitro-6-oxo-1H-[1,4]-thiazino[3,4-a][2]benzazepine-4-carboxylic acid, diphenylmethyl ester

Slurry [4S-[4α,7α(R*),12bβ]]-7-[1,3-dihydro-1,3-dioxo-2H-isoindol-2-yl]-3,4,6,7,8,12b-hexahydro-9-nitro-6-oxo-1H-[1,4]-thiazino[3,4-a][2]benzazepine-4-carboxylic acid, diphenylmethyl ester (0.324mmol) in methanol (6mL) and add hydrazine hydrate (1.0mL of a 1M solution in methanol, 1.0mmol). Stir at room temperature under an argon atmosphere for 20 hours. Dilute with methylene chloride (30mL) and stir for 2 hours. Filter and concentrate *in vacuo*. Take up the residue in methylene chloride and filter again. Wash the filtrate with water, dry (MgSO₄) and evaporate the solvent *in vacuo* to give the title compound.

### Scheme A, Step d: [4S-[4α,7α(R*),12bβ]]-7-[[S-1-[Carbodiphenylmethoxy]-3-phenylpropyl]amino]-3,4,6,7,8,12b-hexahydro-9-nitro-6-oxo-1H-[1,4]-thiazino[3,4-a][2]benzazepine-4-carboxylic acid, diphenylmethyl ester

Dissolve [4S-[4α,7α(R*),12bβ]]-7-amino-3,4,6,7,8,12b-hexahydro-9-nitro-6-oxo-1H-[1,4]-thiazino[3,4-a][2]benzazepine-4-carboxylic acid, diphenylmethyl ester (0.632mmol) and proton sponge (138mg, 0.648mmol) in methylene chloride (5mL). Add a solution of (R)-2-trifluoromethanesulfonyl-4-phenylbutyric acid, diphenylmethyl ester (309mg, 0.648mmol) in methylene chloride (2ml). Stir at room temperature under an argon atmosphere for 26 hours. Purify by flash silica gel chromatography to give the title compound.

### Scheme A, Step e₁: [4S-[4α,7α(R*),12bβ]]-7-[(S-1-Carboxy-3-phenylpropyl]amino]-3,4,6,7,8,12b-hexahydro-9-nitro-6-oxo-1H-[1,4]-thiazino[3,4-a][2]benzazepine-4-carboxylic acid

Dissolve [4S-[4α,7α(R*),12bβ]]-7-[[S-1-[carbodiphenylmethoxy]-3-phenylpropyl]amino]-3,4,6,7,8,12b-hexahydro-9-nitro-6-oxo-1H-[1,4]-thiazino[3,4-a][2]benzazepine-4-carboxylic acid, diphenylmethyl ester (0.045mmol) in methylene chloride (1mL). Add trifluoroacetic acid (1mL) and anisole (0.2mL). Allow to stand for 3 hours under an argon atmosphere. Partition between methylene chloride and 1N hydrochloric acid. Separate the aqueous phase and freeze dry. Purify by High Pressure Liquid Chromatography to give the title compound.

### Example 11

### [4S-[4α,7α(R*),12bβ]]-7-[(S-1-Carboxy-3-phenylpropyl)amino]-3,4,6,7,8,12b-hexahydro-9-nitro-6-oxo-1H-[1,4]-N⁴-trifluoroacetyl-azazino[3,4-a][2]benzazepine-4-carboxylic acid

### Scheme D, step a: N-[2-(1,3-Dihydro-1,3-dioxo-2H-isoindol-2-yl)-1-oxo-3-phenylpropyl]-(S)-3-[(trifluoroacetyl-2-propenyl)amino]-2-amino-propionic acid, methyl ester

Dissolve Nα-(carbobenzyloxy)-β-(amino)-L-alanine, methyl ester (2.27mmol) in anhydrous tetrahydrofuran (15mL). Treat with pyridine (183µL, 2.27mmol) followed by trifluoroacetic anhydride (321µL, 2.27mmol) and stir at ambient temperature for 1 hour. Add additional pyridine (180µL) and trifluoroacetic anhydride (320µL). Allow to stand overnight, partition between ethyl ether and water. Separate the organic phase, dry (MgSO₄) and evaporate the solvent *in vacuo* to give N^{α}-(carbobenzyloxy)-β-(trifluoroacetylamino)-L-alanine, methyl ester.

Suspend sodium hydride (4.8g, 0.2mol) in anhydrous dimethylformamide (100mL), cool to 0°C and place under a nitrogen atmosphere. Add, by dropwise addition, a solution of N^{α}-(carbobenzyloxy)-β-(trifluoroacetylamino)-L-alanine, methyl ester (0.2mol) in dimethylformamide. Stir until evolution of hydrogen ceases. Add, by dropwise addition, a solution of allyl bromide (0.2mol) in dimethylformamide (100mL). Stir overnight at room temperature then carefully quench With saturated ammonium chloride. Extract into ethyl acetate, dry (MgSO₄) and evaporate the solvent *in vacuo.* Purify by silica gel chromatography to give N^{α}-(carbobenzyloxy)-β-(trifluoroacetyl-allylamino)-L-alanine, methyl ester.

Place boron tribromide (215mg, 0.86mmol) in a flask and cool to 0°C. Cautiously add trifluoroacetic acid (5mL) with stirring. Evaporate the solvent to give boron tris(trifluoroacetate).

Dissolve boron tris(trifluoroacetate) (0.3g, 0.86mmol) in trifluoroacetic acid (10mL) and add N^{α}-(carbobenzyloxy)-β-(trifluoroacetyl-allylamino)-L-alanine, methyl ester (105mg, 0.27mmol). Stir under an argon atmosphere for 1 hour then evaporate the solvent in vacuo at room temperature. Add methanol and evaporate repeatedly. Purify by silica gel chromatography to give β-(trifluoroacetyl-allylamino)-L-alanine, methyl ester, hydrochloride.

Dissolve β-(trifluoroacetyl-allylamino)-L-alanine, methyl ester, hydrochloride (104.8g, 0.36mol) in tetrahydrofuran (300mL) then cool to 0°C and add 4-methylmorpholine (88mL, 0.8mol). Add, by dropwise addition, a solution of the N-phthaloyl-(S)-phenylalanine, acid chloride (108.7g, 0.36mol) in tetrahydrofuran (200mL). Allow to warm to room temperature and stir for 3 hours. Filter and concentrate the filtrate in vacuo. Dissolve the residue in ethyl acetate and separate the organic phase. Wash with water then saturated sodium chloride and dry (MgSO₄). Evaporate the solvent in vacuo to give an oil. Purify by silica gel chromatography to give the title compound.

### Scheme D, step b: [S-(R*, R*)]-N-[2-(1,3-Dihydro-1,3-dioxo-2H-isoindol-2-yl)-1-oxo-3-phenylpropyl]-3,4-dihydro-2H-4-trifluoroacetyl-1,4-azazine-3-carboxylic acid, methyl ester

Dissolve N-[2-(1,3-dihydro-1,3-dioxo-2H-isoindol-2-yl)-1-oxo-3-phenylpropyl]-(S)-3-[(trifluoroacetyl-2-propenyl)amino]-2-amino-propionic acid, methyl ester (15.8g, 29.8mmol) in methylene chloride/methanol (10:1, 220mL). Cool to -78°C and sparge with a mixture of ozone/oxygen for approximately 10 minutes until a blue color persists. Sparge with nitrogen for 10 minutes at -78°C to remove excess ozone. Treat with methyl sulfide (60mL, 0.82mol) and allow to warm to room temperature. Stir at room temperature for 2.5 hours, evaporate the solvent in vacuo and dissolve the residue in ethyl acetate (200mL). Wash with water, saturated sodium chloride, dry (MgSO₄) and evaporate the solvent in vacuo to give the intermediate N-[2-(1,3-dihydro-1,3-dioxo-2H-isoindol-2-yl)-1-oxo-3-phenylpropyl]-(S)-3-[(trifluoroacetyl-2-oxoethyl)amino]-2-amino-propionic acid, methyl ester.

Dissolve N-[2-(1,3-dihydro-1,3-dioxo-2H-isoindol-2-yl)-1-oxo-3-phenylpropyl]-(S)-3-[(trifluoroacetyl-2-oxoethyl)amino]-2-amino-propionic acid, methyl ester (15.9g, 29.8mmol) in methylene chloride/trifluoroacetic acid (10:1/330mL). Stir at room temperature for 2.5 hours and evaporate the solvent in vacuo. Purify by silica gel chromatography to give the title compound.

### Scheme D, step c: [4S-[4α, 7α(R*), 12bβ]]-7-[(1,3-Dihydro-1,3-dioxo-2H-isoindol-2-yl)]-3,4,6,7,8,12b-hexahydro-6-oxo-1H-[1,4]-N⁴-trifluoroacetyl-azazino[3,4-a][2]benzazepine-4-carboxylic acid

Dissolve [S-(R*, R*)]-N-[2-(1,3-dihydro-1,3-dioxo-2H-isoindol-2-yl)-1-oxo-3-phenylpropyl]-3,4-dihydro-2H-4-trifluoracetyl-1,4-azazine-3-carboxylic acid, methyl ester (3.04g, 5.9mmol) in methylene chloride (5mL) and add, by dropwise addition, to a previously prepared solution of trifluoromethanesulfonic acid (4.0mL, 45mmol) and trifluoroacetic anhydride (1.0mL, 7.1mmol). Place under a nitrogen atmosphere and stir at room temperature for 123 hours. Pour into a separatory funnel containing ice (200g) and ethyl acetate (200mL). Separate the organic phase, wash with water (3X200mL) and saturated aqueous sodium chloride (100mL). Extract the organic phase with 10% wt. potassium hydrogen carbonate (4X40mL) and water (40mL). Layer the combined basic aqueous phases with ethyl acetate (100mL) and cool in an ice bath. Add, by dropwise addition, 6N hydrochloric acid to adjust the pH to 1 while maintaining the temperature at 5-10°C. Separate the organic phase and extract the aqueous phase with ethyl acetate (3X200mL), wash with saturated sodium chloride and dry (MgSO₄). Evaporate the solvent in vacuo and dry the residue under high vacuum at 56°C for 24 hours to give the title compound.

### Scheme A, step a: [4S-[4α,7α(R*),12bβ]]-7-[1,3-dihydro-1,3-dioxo-2H-isoindol-2-yl]-3,4,6,7,8,12b-hexahydro-9-nitro-6-oxo-1H-[1,4]-N⁴-trifluoroacetyl-azazino[3,4-a][2]benzazepine-4-carboxylic acid and [4S-[4α,7α(R*),12bβ]]-7-[1,3-dihydro-1,3-dioxo-2H-isoindol-2-yl]-3,4,6,7,8,12b-hexahydro-11-nitro-6-oxo-1H-[1,4]-N⁴-trifluoroacetyl-azazino[3,4-a][2]benzazepine-4-carboxylic acid

Dissolve [4S-[4α,7α(R*),12bβ]]-7-[1,3-dihydro-1,3-dioxo-2H-isoindol-2-yl]-3,4,6,7,8,12b-hexahydro-6-oxo-1H-[1,4]-N⁴-trifluoroacetyl-azazino[3,4-a][2]benzazepine-4-carboxylic acid (3.5mmol) in methylene chloride (10mL) and cool to -60°C. Add, by dropwise addition, a solution of nitronium tetrafluoroborate (25mL of a 0.5M solution in sulfolane, 12.5mmol) in methylene chloride (15mL). Warm slowly to 10°C over 34 hours. Partition between methylene chloride (75mL) and water (75mL). Separate the aqueous phase and extract with methylene chloride (50mL). Combine the organic phases, dry (MgSO₄) and evaporate the solvent *in vacuo*. Purify and separate by flash silica gel chromatography to give the 11-nitro title compound and the 9-nitro title compound.

### Scheme A, Step b: [4S-[4α,7α(R*),12bβ]]-7-[1,3-Dihydro-1,3-dioxo-2H-isoindol-2-yl]-3,4,6,7,8,12b-hexahydro-9-nitro-6-oxo-1H-[1,4]-N⁴-trifluoroacetyl-azazino[3,4-a][2]benzazepine-4-carboxylic acid, diphenylmethyl ester

Dissolve [4S-[4α,7α(R*),12bβ]]-7-[1,3-dihydro-1,3-dioxo-2H-isoindol-2-yl]-3,4,6,7,8,12b-hexahydro-9-nitro-6-oxo-1H-[1,4]-N⁴-trifluoroacetyl-azazino[3,4-a][2]benzazepine-4-carboxylic acid (1.50mmol) in methylene chloride (1mL). Add diphenyldiazomethane (291mg, 1.50mmol) and let stand for several days in which time the methylene chloride evaporates. Purify the residue by silica gel chromatography to give the title compound.

### Scheme A, Step c: [4S-[4α,7α(R*),12bβ]]-7-Amino-3,4,6,7,8,12b-hexahydro-9-nitro-6-oxo-1H-[1,4]-N⁴-trifluoroacetyl-azazino[3,4-a][2]benzazepine-4-carboxylic acid, diphenylmethyl ester

Slurry [4S-[4α,7α(R*),12bβ]]-7-[1,3-dihydro-1,3-dioxo-2H-isoindol-2-yl]-3,4,6,7,8,12b-hexahydro-9-nitro-6-oxo-1H-[1,4]-N⁴-trifluoroacetyl-azazino[3,4-a][2]benzazepine-4-carboxylic acid, diphenylmethyl ester (0.324mmol) in methanol (6mL) and add hydrazine hydrate (1.0mL of a 1M solution in methanol, 1.0mmol). Stir at room temperature under an argon atmosphere for 20 hours. Dilute with methylene chloride (30mL) and stir for 2 hours. Filter and concentrate *in vacuo.* Take up the residue in methylene chloride and filter again. Wash the filtrate with water, dry (MgSO₄) and evaporate the solvent *in vacuo* to give the title compound.

### Scheme A, Step d: [4S-[4α,7α(R*),12bβ]]-7-[[S-1-[Carbodiphenylmethoxy]-3-phenylpropyl]amino]-3,4,6,7,8,12b-hexahydro-9-nitro-6-oxo-1H-[1,4]-N⁴-trifluoroacetylazazino[3,4-a][2]benzazepine-4-carboxylic acid, diphenylmethyl ester

Dissolve [4S-[4α,7α(R*),12bβ]]-7-amino-3,4,6,7,8,12b-hexahydro-9-nitro-6-oxo-1H-[1,4]-N⁴-trifluoroacetylazazino[3,4-a][2]benzazepine-4-carboxylic acid, diphenylmethyl ester (0.632mmol) and proton sponge (138mg, 0.648mmol) in methylene chloride (5mL). Add a solution of (R)-2-trifluoromethanesulfonyl-4-phenylbutyric acid, diphenylmethyl ester (309mg, 0.648mmol) in methylene chloride (2ml). Stir at room temperature under an argon atmosphere for 26 hours. Purify by flash silica gel chromatography to give the title compound.

### Scheme A, Step e₁: [4S-[4α,7α(R*),12bβ]]-7-[(S-1-Carboxy-3-phenylpropyl)amino]-3,4,6,7,8,12b-hexahydro-9-nitro-6-oxo-1H-[1,4]-N⁴-trifluoroacetyl-azazino[3,4-a][2]benzazepine-4-carboxylic acid

Dissolve [4S-[4α,7α(R*),12bβ]]-7-[[S-1-[carbodiphenylmethoxy]-3-phenylpropyl]amino]-3,4,6,7,8,12b-hexahydro-9-nitro-6-oxo-1H-[1,4]-N⁴-trifluoroacetylazazino[3,4-a][2]benzazepine-4-carboxylic acid, diphenylmethyl ester (0.045mmol) in methylene chloride (lmL). Add trifluoroacetic acid (1mL) and anisole (0.2mL). Allow to stand for 3 hours under an argon atmosphere. Partition between methylene chloride and 1N hydrochloric acid. Separate the aqueous phase and freeze dry. Purify by High Pressure Liquid Chromatography to give the title compound.

### Example 12

### [4S-[4α,7α(R*),12bβ]]-7-[(S-1-Carboxy-3-phenylpropyl)amino]-3,4,6,7,8,12b-hexahydro-9-nitro-6-oxo-1H-[1,4]-azazino[3,4-a][2]benzazepine-4-carboxylic acid

Dissolve [4S-[4α,7α(R*),12bβ]]-7-[(S-1-carboxy-3-phenylpropyl)amino]-3,4,6,7,8,12b-hexahydro-9-nitro-6-oxo-1H-[1,4]-N⁴-trifluoroacetyl-azazino[3,4-a][2]benzazepine-4-carboxylic acid (2.3mmol) in tetrahydrofuran (10mL) at 0°C then treat with 1N lithium hydroxide (6.75mL). Add ethanol(2-3mL) and stir for 30 minutes at 0°C. Partition between ethyl acetate and water and separate the organic phase. Wash with water, dry (MgSO₄) and evaporate the solvent *in vacuo* to give the title compound.

The following compounds can be prepared by analogous procedures to those described above in Examples 1-12:
[4S-[4α,7α(R*),12bβ]]-7-[(S-1-Carboxy-3-phenylpropyl)amino]-3,4,6,7,8,12b-hexahydro-11-nitro-6-oxo-1H-[1,4]-N⁴-trifluoroacetyl-azazino[3,4-a][2]benzazepine-4-carboxylic acid;
[4S-[4α,7α(R*),12bβ]]-7-[(S-1-Carboxy-3-phenylpropyl)amino]-3,4,6,7,8,12b-hexahydro-9-amino-6-oxo-1H-[1,4]-N⁴-trifluoroacetyl-azazino[3,4-a][2]benzazepine-4-carboxylic acid, dihydrochloride;
[4S-[4α,7α(R*),12bβ]]-7-[(S-1-Carboxy-3-phenylpropyl)amino]-3,4,6,7,8,12b-hexahydro-11-amino-6-oxo-1H-[1,4]-N⁴-trifluoroacetyl-azazino[3,4-a][2]benzazepine-4-carboxylic acid, dihydrochloride;
[4S-[4α,7α(R*),12bβ]]-7-[(S-1-Carboethoxy-3-phenylpropyl)amino]-3,4,6,7,8,12b-hexahydro-11-nitro-6-oxo-1H-[1,4]-N⁴-trifluoroacetyl-azazino[3,4-a][2]benzazepine-4-carboxylic acid;
[4S-[4α,7α(R*),12bβ]]-7-[(S-1-Carboethoxy-3-phenylpropyl)amino]-3,4,6,7,8,12b-hexahydro-11-nitro-6-oxo-1H-[1,4]-N⁴-trifluoroacetyl-azazino[3,4-a][2]benzazepine-4-carboxylic acid;
[4S-[4α,7α(R*),12bβ]]-7-[(S-1-Carboethoxy-3-phenylpropyl)amino]-3,4,6,7,8,12b-hexahydro-9-amino-6-oxo-1H-[1,4]-N⁴-trifluoroacetyl-azazino[3,4-a][2]benzazepine-4-carboxylic acid, dihydrochloride;
[4S-[4α,7α(R*),12bβ]]-7-[(S-1-Carboethoxy-3-phenylpropyl)amino]-3,4,6,7,8,12b-hexahydro-11-amino-6-oxo-1H-[1,4]-N⁴-trifluoroacetyl-azazino[3,4-a][2]benzazepine-4-carboxylic acid, dihydrochloride;
[4S-[4α,7α(R*),12bβ]]-7-[(S-l-Carboxy-3-phenylpropyl)amino]-3,4,6,7,8,12b-hexahydro-11-nitro-6-oxo-1H-[1,4]-azazino[3,4-a][2]benzazepine-4-carboxylic acid;
[4S-[4α,7α(R*),12bβ]]-7-[(S-1-Carboxy-3-phenylpropyl)amino]-3,4,6,7,8,12b-hexahydro-9-amino-6-oxz-1H-[1,4]-azazino[3,4-a][2]benzazepine-4-carboxylic acid, dihydrochloride;
[4S-[4α,7α(R*),12bβ]]-7-[(S-1-Carboxy-3-phenylpropyl)amino]-3,4,6,7,8,12b-hexahydro-11-amino-6-oxo-1H-[1,4]-azazino[3,4-a][2]benzazepine-4-carboxylic acid, dihydrochloride;
[4S-[4α,7α(R*),12bβ]]-7-[(S-1-Carboethoxy-3-phenylpropyl)amino]-3,4,6,7,8,12b-hexahydro-11-nitro-6-oxo-1H-[1,4]-azazino[3,4-a][2]benzazepine-4-carboxylic acid;
[4S-[4α,7α(R*),12bβ]]-7-[(S-1-Carboethoxy-3-phenylpropyl)amino]-3,4,6,7,8,12b-hexahydro-11-nitro-6-oxo-1H-[1,4]-azazino[3,4-a][2]benzazepine-4-carboxylic acid;
[4S-[4α,7α(R*),12bβ]]-7-[(S-1-Carboethoxy-3-phenylpropyl)amino]-3,4,6,7,8,12b-hexahydro-9-amino-6-oxo-1H-[1,4]-azazino[3,4-a][2]benzazepine-4-carboxylic acid, dihydrochloride;
[4S-[4α,7α(R*),12bβ]]-7-[(S-1-Carboethoxy-3-phenylpropyl)amino]-3,4,6,7,8,12b-hexahydro-11-amino-6-oxo-1H-[1,4]-azazino[3,4-a][2]benzazepine-4-carboxylic acid, dihydrochloride;
[4S-[4α,7α(R*),12bβ]]-7-[(S-1-Carboxy-3-phenylpropyl)amino]-3,4,6,7,8,12b-hexahydro-11-nitro-6-oxo-1H-[1,4]-thiazino[3,4-a][2]benzazepine-4-carboxylic acid;
[4S-[4α,7α(R*),12bβ]]-7-[(S-1-Carboxy-3-phenylpropyl)amino]-3,4,6,7,8,12b-hexahydro-9-amino-6-oxo-1H-[1,4]-thiazino[3,4-a][2]benzazepine-4-carboxylic acid, dihydrochloride;
[4S-[4α,7α(R*),12bβ]]-7-[(S-1-Carboxy-3-phenylpropyl)amino]-3,4,6,7,8,12b-hexahydro-11-amino-6-oxo-1H-[1,4]-thiazino[3,4-a][2]benzazepine-4-carboxylic acid, dihydrochloride;
[4S-[4α,7α(R*),12bβ]]-7-[(S-1-Carboethoxy-3-phenylpropyl)amino]-3,4,6,7,8,12b-hexahydro-11-nitro-6-oxo-1H-[1,4]-thiazino[3,4-a][2]benzazepine-4-carboxylic acid;
[4S-[4α,7α(R*),12bβ]]-7-[(S-1-Carboethoxy-3-phenylpropyl)amino]-3,4,6,7,8,12b-hexahydro-11-nitro-6-oxo-1H-[1,4]-thiazino[3,4-a][2]benzazepine-4-carboxylic acid;
[4S-[4α,7α(R*),12bβ]]-7-[(S-1-Carboethoxy-3-phenylpropyl)amino]-3,4,6,7,8,12b-hexahydro-9-amino-6-oxo-1H-[1,4]-thiazino[3,4-a][2]benzazepine-4-carboxylic acid, dihydrochloride;
[4S-[4α,7α(R*),12bβ]]-7-[(S-1-Carboethoxy-3-phenylpropyl)amino]-3,4,6,7,8,12b-hexahydro-11-amino-6-oxo-1H-[1,4]-thiazino[3,4-a][2]benzazepine-4-carboxylic acid, dihydrochloride;
[4S-[4α,7α(R*),12bβ]]-7-[(S-1-Carboxy-3-phenylpropyl)amino]-3,4,6,7,8,12b-hexahydro-11-nitro-6-oxo-1H-[1,4]-oxazino[3,4-a][2]benzazepine-4-carboxylic acid;
[4S-[4α,7α(R*),12bβ]]-7-[(S-1-Carboxy-3-phenylpropyl)amino]-3,4,6,7,8,12b-hexahydro-9-amino-6-oxo-1H-[1,4]-oxazino[3,4-a][2]benzazepine-4-carboxylic acid, dihydrochloride;
[4S-[4α,7α(R*),12bβ]]-7-[(S-1-Carboxy-3-phenylpropyl)amino]-3,4,6,7,8,12b-hexahydro-11-amino-6-oxo-1H-[1,4]-oxazino[3,4-a][2]benzazepine-4-carboxylic acid, dihydrochloride;
[4S-[4α,7α(R*),12bβ]]-7-[(S-1-Carboethoxy-3-phenylpropyl)amino]-3,4,6,7,8,12b-hexahydro-11-nitro-6-oxo-1H-[1,4]-oxazino[3,4-a][2]benzazepine-4-carboxylic acid;
[4S-[4α,7α(R*),12bβ]]-7-[(S-1-Carboethoxy-3-phenylpropyl)amino]-3,4,6,7,8,12b-hexahydro-11-nitro-6-oxo-1H-[1,4]-oxazino[3,4-a][2]benzazepine-4-carboxylic acid;
[4S-[4α,7α(R*),12bβ]]-7-[(S-1-Carboethoxy-3-phenylpropyl)amino]-3,4,6,7,8,12b-hexahydro-9-amino-6-oxo-1H-[1,4]-oxazino[3,4-a][2]benzazepine-4-carboxylic acid, dihydrochloride;
[4S-[4α,7α(R*),12bβ]]-7-[(S-1-Carboethoxy-3-phenylpropyl)amino]-3,4,6,7,8,12b-hexahydro-11-amino-6-oxo-1H-[1,4]-oxazino[3,4-a][2]benzazepine-4-carboxylic acid, dihydrochloride.

In a further embodiment, the present invention provides pharmaceutical compositions useful in inhibiting ACE in a patient in need thereof comprising a compound of formula (1).

As used herein, the term "patient" refers to warm-blooded animals or mammals, including mice, rats and humans. A patient is in need of treatment to inhibit ACE when it would be beneficial to the patient to reduce the physiological effect of circulating angiotensin II. For example, a patient is in need of treatment to inhibit ACE when the patient is suffering from hypertension, chronic congestive heart failure, hyperaldosteronemia or cognitive disorders. Inhibition of ACE reduces levels of angiotensin II and thus inhibits the vasopressor, hypertensive and hyper-aldosteronemic effects caused thereby.

An effective ACE inhibitory amount of a compound of formula (1) is that amount which is effective in inhibiting ACE in a patient in need thereof which results, for example, in a hypotensive effect.

An effective ACE inhibitory dose can be readily determined by the use of conventional techniques and by observing results obtained under analogous circumstances. In determining the effective dose, a number of factors are considered including, but not limited to: the species of patient; its size, age, and general health; the specific disease involved; the degree of or involvement or the severity of the disease; the response of the individual patient; the particular compound administered; the mode of administration; the bioavailability characteristics of the preparation administered; the dose regimen selected; and the use of concomitant medication.

An effective ACE inhibitory amount of a compound of formula (1) will generally vary from about 0.01 milligram per kilogram of body weight per day (mg/kg/day) to about 20 mg/kg/day. A daily dose of from about 0.1 mg/kg to about 10 mg/kg is preferred.

The pharmaceutical compositions comprising the compounds of formula (1) can be administered in any form or mode which makes the compound bioavailable in effective amounts, including oral and parenteral routes. For example, they can be administered orally, subcutaneously, intramuscularly, intravenously, transdermally, intranasally, rectally, and the like. Oral administration is generally preferred. One skilled in the art of preparing formulations can readily select the proper form and mode of administration depending upon the disease state to be treated, the stage of the disease, and other relevant circumstances.

Compounds of formula (1) can be administered in the form of pharmaceutical compositions or medicaments which are made by combining the compounds of formula (1) with pharmaceutically acceptable carriers or excipients, the proportion and nature of which are determined by the chosen route of administration, and standard pharmaceutical practice.

In another embodiment, the present invention provides compositions comprising a compound of formula (1) in admixture or otherwise in association with one or more inert carriers. These compositions are useful, for example, as assay standards, as convenient means of making bulk shipments, or as pharmaceutical compositions. An assayable amount of a compound of formula (1) is an amount which is readily measurable by standard assay procedures and techniques as are well known and appreciated by those skilled in the art. Assayable amounts of a compound of formula (1) will generally vary from about 0.001% to about 75% of the composition by weight. Inert carriers can be any material which does not degrade or otherwise covalently react with a compound of formula (1). Examples of suitable inert carriers are water; aqueous buffers, such as those which are generally useful in High Performance Liquid Chromatography (HPLC) analysis; organic solvents, such as acetonitrile, ethyl acetate, hexane and the like; and pharmaceutically acceptable carriers or excipients.

More particularly, the present invention provides pharmaceutical compositions comprising an effective amount of a compound of formula (1) in admixture or otherwise in association with one or more pharmaceutically acceptable carriers or excipients.

The pharmaceutical compositions or medicaments are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The pharmaceutical compositions may be administered orally, for example, with an inert diluent or with an edible carrier. They may be enclosed in gelatin capsules or compressed into tablets. For the purpose of oral therapeutic administration, the compounds of formula (1) may be incorporated with excipients and used in the form of tablets, troches, capsules, elixirs, suspensions, syrups, wafers, chewing gums and the like. These preparations should contain at least 4% of the compound of formula (1), the active ingredient, but may be varied depending upon the particular form and may conveniently be between 4% to about 70% of the weight of the unit. The amount of the active ingredient present in compositions is such that a unit dosage form suitable for administration will be obtained.

The tablets, pills, capsules, troches and the like may also contain one or more of the following adjuvants: binders, such as microcrystalline cellulose, gum tragacanth or gelatin; excipients, such as starch or lactose, disintegrating agents such as alginic acid, Primogel, corn starch and the like; lubricants, such as magnesium stearate or Sterotex; glidants, such as colloidal silicon dioxide; and sweetening agents, such as sucrose or saccharin may be added or flavoring agents, such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active ingredient, sucrose as a sweetening agent and certain preservatives, dyes and colorings and flavors. Materials used in preparing these various compositions should be pharmaceutically pure and non-toxic in the amounts used.

For the purpose of parenteral administration, the compounds of formula (1) may be incorporated into a solution or suspension. These preparations should contain at least 0.1% of a compound of the invention, but may be varied to be between 0.1 and about 50% of the weight thereof. The amount of the active ingredient present in such compositions is such that a suitable dosage will be obtained.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of toxicity such as sodium chloride or dextrose. The parenteral preparation can be enclosed in ampules, disposable syringes or multiple dose vials made of glass or plastic.

As with any group of structurally related compounds which possess a particular generic utility, certain groups and configurations are preferred for compounds of formula (1) in their end-use application.

The compounds of formula (1) wherein X is amino and Y is hydrogen are generally preferred.

It is, of course, understood that the compounds of formula (1) exist in particular isomeric configurations including structural as well as stereo isomers. It is further understood that the present invention encompasses those compounds in the particular isomeric configurations depicted by the structure of formula (1) which utilizes standard techniques and conventions for drawing isomeric structures.

The following specific compounds of formula (1) are particularly preferred in the end-use application of the compounds of the present invention:
[4S-[4α,7α(R*),12bβ]]-7-[(S-1-Carboxy-3-phenylpropyl)amino]-1,2,3,4,6,7,8,12b-octahydro-9-nitro-6-oxopyrido[2,1-a][2]benzazepine-4-carboxylic acid;
[4S-[4α,7α(R*),12bβ]]-9-Amino-7-[(S-1-carboxy-3-phenylpropyl)amino]-1,2,3,4,6,7,8,12b-octahydro-6-oxopyrido[2,1-a][2]benzazepine-4-carboxylic acid, dihydrochloride;
[4S-[4α,7α(R*),12bβ]]-7-[(S-1-Carboxy-3-phenylpropyl)amino]-1,2,3,4,6,7,8,12b-octahydro-11-nitro-6-oxopyrido[2,1-a][2]benzazepine-4-carboxylic acid;
[4S-[4α,7α(R*),12bβ]]-11-Amino-7-[(S-1-carboxy-3-phenylpropyl)amino]-1,2,3,4,6,7,8,12b-octahydro-6-oxopyrido[2,1-a][2]benzazepine-4-carboxylic acid, dihydrochloride;
[4S-[4α,7α(R*),12bβ]]-7-[(S-1-Carbethoxy-3-phenylpropyl)amino]-1,2,3,4,6,7,8,12b-octahydro-9-nitro-6-oxopyrido[2,1-a][2]benzazepine-4-carboxylic acid;
[4S-[4α,7α(R*),12bβ]]-9-Amino-7-[(1-carbethoxy-3-phenylpropyl)amino]-1,2,3,4,6,7,8,12b-octahydro-6-oxopyrido[2,1-a][2]benzazepine-4-carboxylic acid dihydrochloride;
[4S-[4α,7α(R*),12bβ]]-7-[(S-1-Carbethoxy-3-phenylpropyl)amino]-1,2,3,4,6,7,8,12b-octahydro-11-nitro-6-oxopyrido[2,1-a][2]benzazepine-4-carboxylic acid; [4S-[4α,7α(R*),12bβ]]-11-Amino-7-[(S-1-carbethoxy-3-phenylpropyl)amino]-1,2,3,4,6,7,8,12b-octahydro-6-oxopyrido[2,1-a][2]benzazepine-4-carboxylic acid, dihydrochloride;

The utility of the compounds of formula (1) may be demonstrated by monitoring the inhibition of ACE *in vitro* using the spectrophotometric substrate described by Holmquist et al. [*Anal.Biochem.* 95, 540-548 (1979)] and the buffer system described by Ryan [*Methods of Enzymatic Analysis*, 3rd ed., H. U. Bergmeyer, editor; vol. *V*, Verlag Chemie, Weinheim, 1983, pp. 20-34].

The amino or nitro substitued compounds of the present invention possess an unexpectedly prolonged duration of activity as compared to other ACE inhibitors similar in structure.

## Claims

1. A compound of the formula 1 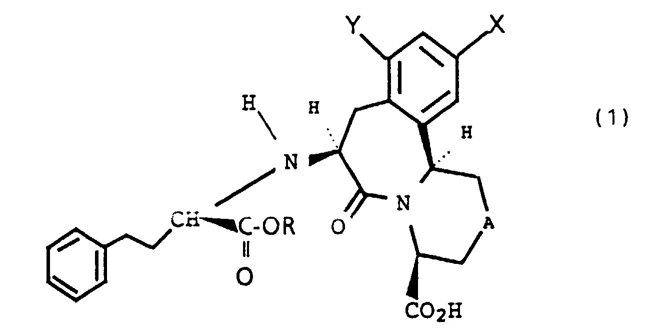 wherein
A is methylene, oxygen, sulfur or N-B wherein B is R₁ or COR₂ wherein R₁ is hydrogen, a C₁-C₄ alkyl or an Ar-Z-group wherein Ar is aryl and Z is a C₀-C₄ alkyl and R₂ is a -CF₃, a C₁-C₁₀ alkyl or an Ar-Z group;
R is hydrogen or a C₁-C₄ alkyl; and
X and Y are each independently hydrogen, nitro or amino, with the proviso that one of X and Y is hydrogen and one of X and Y is other than hydrogen; and
pharmaceutically acceptable salts thereof.

2. A compound of Claim 1 wherein Y is nitro and X is hydrogen.

3. A compound of Claim 1 wherein Y is amino and X is hydrogen.

4. A compound of Claim 1 wherein X is nitro and Y is hydrogen.

5. A compound of Claim 1 wherein X is amino and Y is hydrogen.

6. A compound of Claim 1 wherein A is methylene.

7. A compound of Claim 1 wherein A is oxygen.

8. A compound of Claim 1 wherein A is sulfur.

9. A compound of Claim i wherein A is -N-B-.

10. A compound of Claim 1 wherein the compound is
[4S-[4α,7α(R*),12bβ]]-7-[(S-1-Carboxy-3-phenylpropyl)amino]-1,2,3,4,6,7,8,12b-octahydro-9-nitro-6-oxopyrido[2,1-a][2]benzazepine-4-carboxylic acid ; or
[4S-[4α,7α(R*),12bβ]]-9-Amino-7-[(S-1-carboxy-3-phenylpropyl)amino]-1,2,3,4,6,7,8,12b-octahydro-6-oxopyrido[2,1-a][2]benzazepine-4-carboxylic acid ; or
[4S-[4α,7α(R*),12bβ]]-7-[(S-1-Carboxy-3-phenylpropyl)amino]-1,2,3,4,6,7,8,12b-octahydro-11-nitro-6-oxopyrido[2,1-a][2]benzazepine-4-carboxylic acid ; or
[4S-[4α,7α(R*),12bβ]]-11-Amino-7-[(S-1-carboxy-3-phenylpropyl)amino]-1,2,3,4,6,7,8,12b-octahydro-6-oxopyrido[2,1-a][2]benzazepine-4-carboxylic acid ; or
[4S-[4α,7α(R*),12bβ]]-7-[(S-1-Carbethoxy-3-phenylpropyl)amino]-1,2,3,4,6,7,8,12b-octahydro-9-nitro-6-oxopyrido[2,1-a][2]benzazepine-4-carboxylic acid ; or
[4S-[4α,7α(R*),12bβ]]-9-Amino-7-[(1-carbethoxy-3-phenylpropyl)amino]-1,2,3,4,6,7,8,12b-octahydro-6-oxopyrido[2,1-a][2]benzazepine-4-carboxylic acid ; or
[4S-[4α,7α(R*),12bβ]]-7-[(S-1-Carbethoxy-3-phenylpropyl)amino]-1,2,3,4,6,7,8,12b-octahydro-11-nitro-6-oxopyrido[2,1-a][2]benzazepine-4-carboxylic acid ; or
[4S-[4α,7α(R*),12bβ]]-11-Amino-7-[(S-1-carbethoxy-3-phenylpropyl)amino]-1,2,3,4,6,7,8,12b-octahydro-6-oxopyrido[2,1-a][2]benzazepine-4-carboxylic acid.

11. A pharmaceutical composition comprising a compound of any one of Claims 1 to 10 in admixture or otherwise in association with one or more pharmaceutically acceptable carriers or excipients.

12. A pharmaceutical composition according to Claim 11 useful in inhibiting ACE.

13. A pharmaceutical composition according to Claim 11 useful in treating hypertension.

14. A pharmaceutical composition according to Claim 11 useful in treating chronic congestive heart failure.

15. A composition comprising an assayable amount of a compound of Claim 1 in admixture or otherwise in association with an inert carrier.

16. A process for preparing a compound as defined in any one of Claims 1-10 of formula 1' 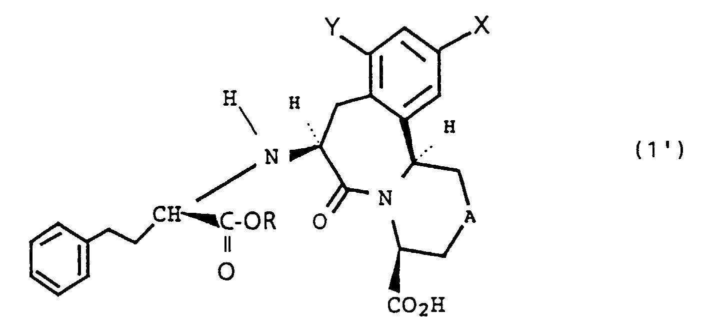 wherein
A is methylene, oxygen, sulfur or N-B wherein B is R₁ or COR₂ wherein R₁ is a C₁-C₄ alkyl or an Ar-Z- group wherein Ar is aryl and Z is a C₀-C₄ alkyl and R₂ is a -CF₃, a C₁-C₁₀ alkyl or an Ar-Z group;
R is hydrogen; and
X and Y are each independently hydrogen or nitro, with the proviso that one of X and Y is hydrogen and one of X and Y is other than hydrogen; and
the pharmaceutically acceptable salts thereof,
comprising reacting a compound of formula 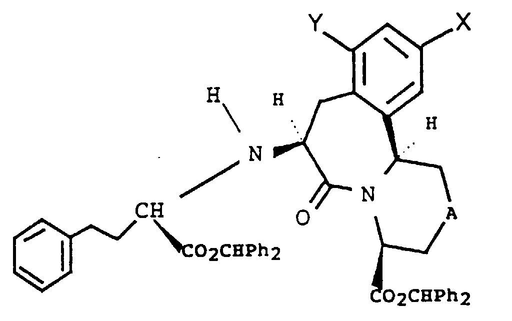 wherein Ph is phenyl with an acid or a reducing agent.

17. A process for preparing a compound as defined in any one of claims 1-10 of formula 1b 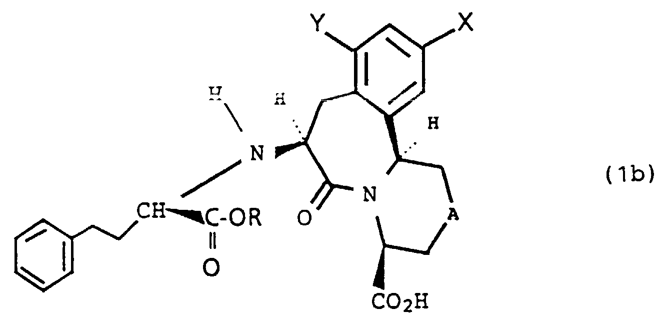 wherein
A is methylene, oxygen, sulfur or N-B wherein B is R₁ or COR₂ wherein R₁ is a C₁-C₄ alkyl or an Ar-Z- group wherein Ar is aryl and Z is a C₀-C₄ alkyl and R₂ is a -CF₃, a C₁-C₁₀ alkyl or an Ar-Z group;
R is a C₁-C₄ alkyl; and
X and Y are each independently hydrogen or nitro, with the proviso that one of X and Y is hydrogen and one of X and Y is other than hydrogen; and
the pharmaceutically acceptable salts thereof,
comprising reacting a compound of formula 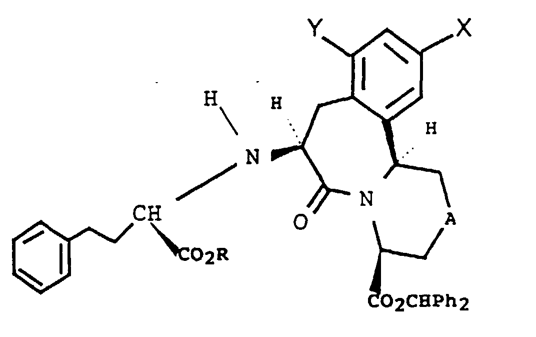 wherein Ph is phenyl with an acid or a reducing agent.

18. A process for preparing a compound as defined in any one of claims 1-10 of formula 1c 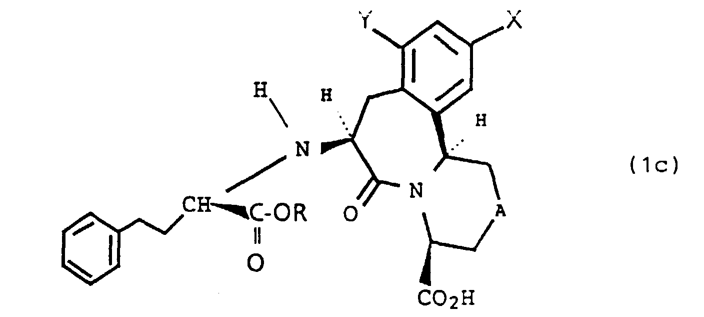 wherein
A is N-H
R is hydrogen or a C₁-C₄ alkyl; and
X and Y are each independently hydrogen, nitro or amino, with the proviso that one of X and Y is hydrogen and one of X and Y is other than hydrogen; and
the pharmaceutically acceptable salts thereof,
comprising reacting a compound of formula 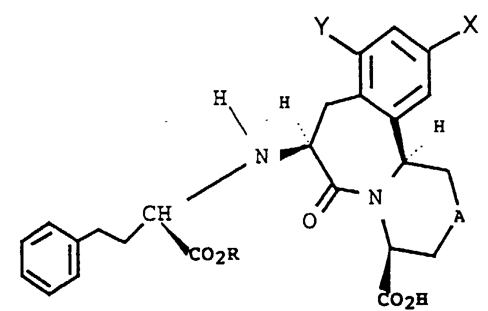 wherein A is N-COR₂ wherein R₂ is a -CF₃, a C₁-C₁₀ alkyl or an Ar-Z group wherein Ar is aryl and Z is a C₀-C₄ alkyl with a base.

19. A process for the preparation of a pharmaceutical composition as defined in any one of claims 11-14 comprising combining a compound according to any one of Claims 1 to 10 with one or more pharmaceutically acceptable carriers or excipients.

## Patentansprüche

1. Verbindung der Formel 1 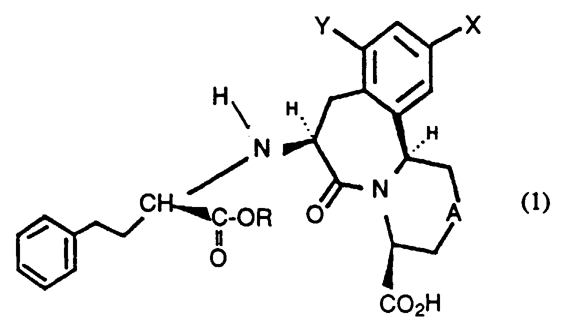 wobei
A für eine Methylengruppe, ein Sauerstoff-, ein Schwefelatom oder N-B steht, wobei B R₁ oder COR₂ bedeutet, wobei R₁ für ein Wasserstoffatom, einen C₁-C₄-Alkylrest oder einen Ar-Z-Rest steht, wobei Ar einen Arylrest und Z einen C₀-C₄-Alkylrest bedeutet, und R₂ für eine -CF₃-Gruppe, einen C₁-C₁₀-Alkyl- oder Ar-Z-Rest steht,
R ein Wasserstoffatom oder einen C₁-C₄-Alkylrest darstellt, und
X und Y jeweils unabhängig voneinander ein Wasserstoffatom, eine Nitro- oder Aminogruppe bedeuten, mit der Maßgabe, daß einer der Reste X und Y ein Wasserstoffatom darstellt und einer der Reste X und Y von einem Wasserstoffatom verschieden ist,
sowie die pharmazeutisch verträglichen Salze davon.

2. Verbindung nach Anspruch 1, wobei Y für eine Nitrogruppe und X für ein Wasserstoffatom steht.

3. Verbindung nach Anspruch 1, wobei Y für einen Aminorest und X für ein Wasserstoffatom steht.

4. Verbindung nach Anspruch 1, wobei X für eine Nitrogruppe und Y für ein Wasserstoffatom steht.

5. Verbindung nach Anspruch 1, wobei X für einen Aminorest und Y für ein Wasserstoffatom steht.

6. Verbindung nach Anspruch 1, wobei A für eine Methylengruppe steht.

7. Verbindung nach Anspruch 1, wobei A für ein Sauerstoffatom steht.

8. Verbindung nach Anspruch 1, wobei A für ein Schwefelatom steht.

9. Verbindung nach Anspruch 1, wobei A für -N-B- steht.

10. Verbindung nach Anspruch 1, wobei die Verbindung
[4S-[4α,7α(R*),12bβ]]-7-[(S-1-Carboxy-3-phenylpropyl)amino]-1,2,3,4,6,7,8,12b-oktahydro-9-nitro-6-oxopyrido[2,1-a][2]benzazepin-4-carbonsäure oder
[4S-[4α,7α(R*),12bβ]]-9-Amino-7-[(S-1-carboxy-3-phenylpropyl)amino]-1,2,3,4,6,7,8,12b-oktahydro-6-oxopyrido[2,1-a][2]benzazepin-4-carbonsäure oder
[4S-[4α,7α(R*),12bβ]]-7-[(S-1-Carboxy-3-phenylpropyl)amino]-1,2,3,4,6,7,8,12b-oktahydro-11-nitro-6-oxopyrido[2,1-a][2]benzazepin-4-carbonsäure oder
[4S-[4α,7α(R*),12bβ]]-11-Amino-7-[(S-1-carboxy-3-phenylpropyl)amino]-1,2,3,4,6,7,8,12b-oktahydro-6-oxopyrido[2,1-a][2]benzazepin-4-carbonsäure oder
[4S-[4α,7α(R*),12bβ]]-7-[(S-1-Carbethoxy-3-phenylpropyl)amino]-1,2,3,4,6,7,8,12b-oktahydro-9-nitro-6-oxopyrido[2,1-a][2]benzazepin-4-carbonsäure oder
[4S-[4α,7α(R∗),12bβ]]-9-Amino-7-[(1-carbethoxy-3-phenylpropyl)amino]-1,2,3,4,6,7,8,12b-oktahydro-6-oxopyrido[2,1-a][2]benzazepin-4-carbonsäure oder
[4S-[4α,7α(R*),12bβ]]-7-[(S-1-Carbethoxy-3-phenylpropyl)amino]-1,2,3,4,6,7,8,12b-oktahydro-11-nitro-6-oxopyrido[2,1-a][2]benzazepin-4-carbonsäure oder
[4S-[4α,7α(R*),12bβ]]-11-Amino-7-[(S-1-carbethoxy-3-phenylpropyl)amino]-1,2,3,4,6,7,8,12b-oktahydro-6-oxopyrido[2,1-a][2]benzazepin-4-carbonsäure
ist.

11. Arzneimittel, umfassend eine Verbindung nach einem der Ansprüche 1 bis 10 im Gemisch oder anderweitig in Verbindung mit einem oder mehreren pharmazeutisch verträglichen Trägern oder Excipienten.

12. Arzneimittel nach Anspruch 11, das bei der ACE-Hemmung nützlich ist.

13. Arzneimittel nach Anspruch 11, das bei der Behandlung von Bluthochdruck nützlich ist.

14. Arzneimittel nach Anspruch 11, das bei der Behandlung von chronischer Herzinsuffizienz nützlich ist.

15. Zusammensetzung, umfassend eine nachweisbare Menge einer Verbindung nach Anspruch 1 im Gemisch oder anderweitig in Verbindung mit einem inerten Träger.

16. Verfahren zur Herstellung einer Verbindung der Formel 1', nach einem der Ansprüche 1 - 10, 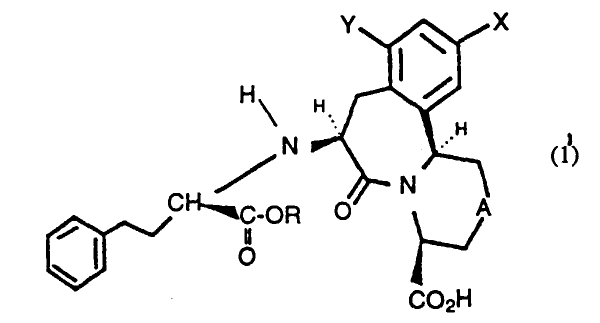 wobei
A für eine Methylengruppe, ein Sauerstoff-, ein Schwefelatom oder N-B steht, wobei B R₁ oder COR₂ bedeutet, wobei R₁ für einen C₁-C₄-Alkylrest oder einen Ar-Z-Rest steht, wobei Ar einen Arylrest und Z einen C₀-C₄-Alkylrest bedeutet, und R₂ für eine -CF₃-Gruppe, einen C₁-C₁₀-Alkyl- oder Ar-Z-Rest steht,
R ein Wasserstoffatom darstellt, und
X und Y jeweils unabhängig voneinander ein Wasserstoffatom oder eine Nitrogruppe bedeuten, mit der Maßgabe, daß einer der Reste X und Y ein Wasserstoffatom darstellt und einer der Reste X und Y von einem Wasserstoffatom verschieden ist,
sowie die pharmazeutisch verträglichen Salze davon, umfassend Umsetzen einer Verbindung der Formel, 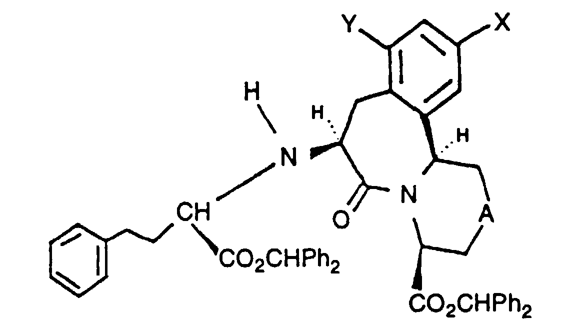 wobei Ph für eine Phenylgruppe steht, mit einer Säure oder einem Reduktionsmittel.

17. Verfahren zur Herstellung einer Verbindung der Formel 1b, nach einem der Ansprüche 1 - 10, 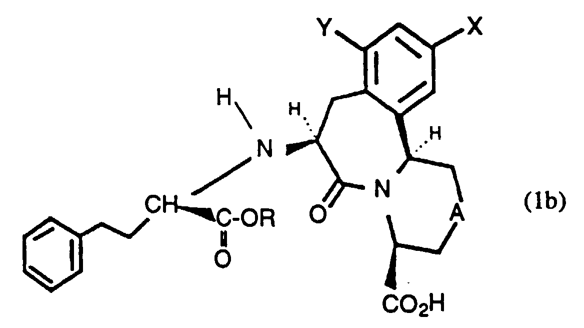 wobei
A für eine Methylengruppe, ein Sauerstoff-, ein Schwefelatom oder N-B steht, wobei B R₁ oder COR₂ bedeutet, wobei R₁ für einen C₁-C₄-Akylrest oder einen Ar-Z-Rest steht, wobei Ar einen Arylrest und Z einen C₀-C₄-Alkylrest bedeutet, und R₂ für eine -CF₃-Gruppe, einen C₁-C₁₀-Alkyl- oder Ar-Z-Rest steht,
R einen C₁-C₄-Alkylrest darstellt, und
X und Y jeweils unabhängig voneinander ein Wasserstoffatom oder eine Nitrogruppe bedeuten, mit der Maßgabe, daß eines von X und Y ein Wasserstoffatom darstellt und eines von X und Y von einem Wasserstoffatom verschieden ist,
sowie die pharmazeutisch verträglichen Salze davon, umfassend Umsetzen einer Verbindung der Formel, 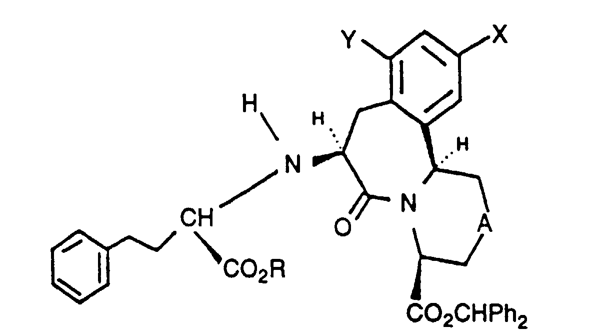 wobei Ph für einen Phenylrest steht, mit einer Säure oder einem Reduktionsmittel.

18. Verfahren zur Herstellung einer Verbindung der Formel 1c, nach einem der Ansprüche 1 - 10, 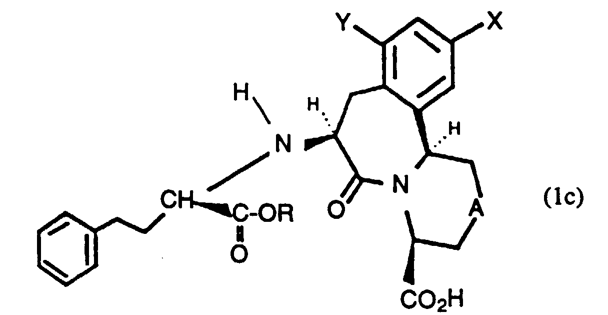 wobei
A für N-H steht,
R ein Wasserstoffatom oder einen C₁-C₄-Alkylrest darstellt, und
X und Y jeweils unabhängig voneinander ein Wasserstoffatom, eine Nitro- oder eine Aminogruppe bedeuten, mit der Maßgabe, daß einer der Reste X und Y ein Wasserstoffatom darstellt und einer der Reste X und Y von einem Wasserstoffatom verschieden ist,
sowie die pharmazeutisch verträglichen Salze davon, umfassend Umsetzen einer Verbindung der Formel, 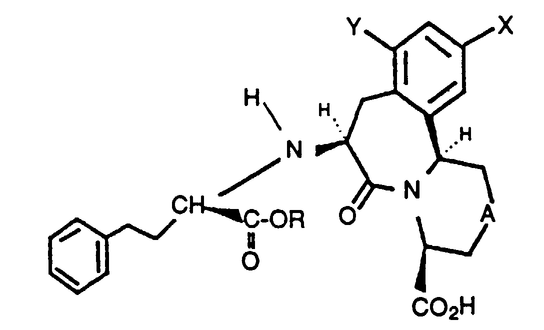 wobei A für N-COR₂ steht, wobei R₂ für eine -CF₃-Gruppe, einen C₁-C₁₀-Alkyl- oder Ar-Z-Rest steht, wobei Ar einen Arylrest und Z einen C₀-C₄-Alkylrest bedeuten, mit einer Base.

19. Verfahren zur Herstellung eines Arzneimittels, nach einem der Ansprüche 11 - 14, umfassend Vereinigen einer Verbindung nach einem der Ansprüche 1 bis 10 mit einem oder mehreren pharmazeutisch verträglichen Trägern oder Excipienten.

## Revendications

1. Composé de formule 1 : 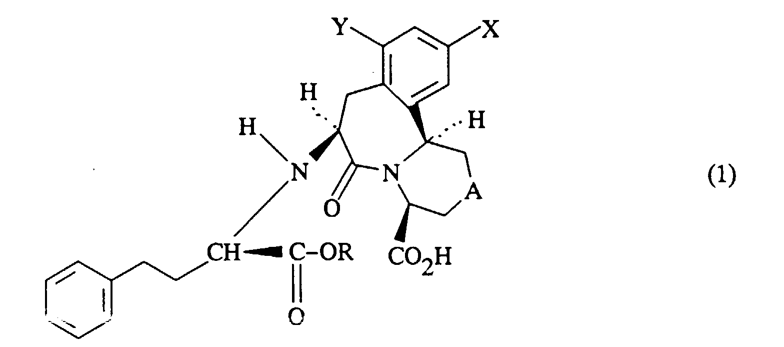 où
A est un radical méthylène, oxygène, soufre ou N-B où B est R₁ ou COR₂ où R₁ est un radical hydrogène, un alkyle en C₁-C₄ ou un groupe Ar-Z où Ar est un aryle et Z est un alkyle en C₀-C₄ et R₂ est un radical -CF₃, un alkyle en C₁-C₁₀ ou un groupe Ar-Z ;
R est un hydrogène ou un alkyle en C₁-C₄ ; et
X et Y sont chacun indépendamment un radical hydrogène, azote ou amine, pour autant que soit X soit Y soit un hydrogène et que soit X soit Y ne soit pas un hydrogène ; et
ses sels pharmaceutiquement acceptables.

2. Composé selon la revendication 1, où Y est un azote et X est un hydrogène.

3. Composé selon la revendication 1, où Y est une amine et X est un hydrogène.

4. Composé selon la revendication 1, où X est un azote et Y est un hydrogène.

5. Composé selon la revendication 1, où X est une amine et Y est un hydrogène.

6. Composé selon la revendication 1, où A est un méthylène.

7. Composé selon la revendication 1, où A est un oxygène.

8. Composé selon la revendication 1, où A est un soufre.

9. Composé selon la revendication 1, où A est -N-B-.

10. Composé selon la revendication 1, où le composé est l'acide
[4S-[4α,7α(R*),12bβ]]-7-[(S-1-carboxy-3-phénylpropyl)amino]-1,2,3,4,6,7,8,12b-octahydro-9-nitro-6-oxypyrido[2,1-a][2]benzazépine-4-carboxylique; ou
l'acide [4S-[4α,7α(R*),12bβ]]-9-amino-7-[(S-1-carboxy-3-phénylpropyl)amino]-1,2,3,4,6,7,8,12b-octahydro-6-oxopyrido[2,1-a][2]benzazépine-4-carboxylique ; ou
l'acide [4S-[4α,7α(R*),12bβ]]-7-[(S-1-carboxy-3-phénylpropyl)amino]-1,2,3,4,6,7,8,12b-octahydro-11-nitro-6-oxopyrido[2,1-a][2]benzazépine-4-carboxylique ; ou
l'acide [4S-[4α,7α(R*),12bβ]]-11-amino-7-[(S-1-carboxy-3-phénylpropyl)amino]-1,2,3,4,6,7,8,12b-octahydro-6-oxopyrido[2,1-a][2]benzazépine-4-carboxylique ; ou
l'acide [4S-[4α,7α(R*),12bβ]]-7-[(S-1-carbéthoxy-3-phénylpropyl)amino]-1,2,3,4,6,7,8,12b-octahydro-9-nitro-6-oxopyrido[2,1-a][2]benzazépine-4-carboxylique ; ou
l'acide [4S-[4α,7α(R*),12bβ]]-9-amino-7-[(1-carbéthoxy-3-phénylpropyl)amino]-1,2,3,4,6,7,8,12b-octahydro-6-oxopyrido[2,1-a][2]benzazépine-4-carboxylique ; ou
l'acide [4S-[4α,7α(R*),12bβ]]-7-[(S-1-carbéthoxy-3-phénylpropyl)amino]-1,2,3,4,6,7,8,12b-octahydro-11-nitro-6-oxopyrido[2,1-a][2]benzazépine-4-carboxylique ; ou
l'acide [4S-[4α,7α(R*),12bβ]]-11-amino-7-[(S-1-carbéthoxy-3-phénylpropyl)amino]-1,2,3,4,6,7,8,12b-octahydro-6-oxopyrido[2,1-a][2]benzazépine-4-carboxylique.

11. Composition pharmaceutique comprenant un composé selon l'une quelconque des revendications 1 à 10 en mélange ou association autre avec un ou plusieurs véhicules ou excipients pharmaceutiquement acceptables.

12. Composition pharmaceutique selon la revendication 11, utile dans l'inhibition de l'enzyme de conversion de l'angiotensine.

13. Composition pharmaceutique selon la revendication 11, utile dans le traitement de l'hypertension.

14. Composition pharmaceutique selon la revendication 11, utile dans le traitement de l'insuffisance cardiaque congestive chronique.

15. Composition comprenant une quantité dosable d'un composé selon la revendication 1 en mélange ou association autre avec un véhicule inerte.

16. Procédé de préparation d'un composé selon l'une quelconque des revendications 1 à 10, de formule 1': 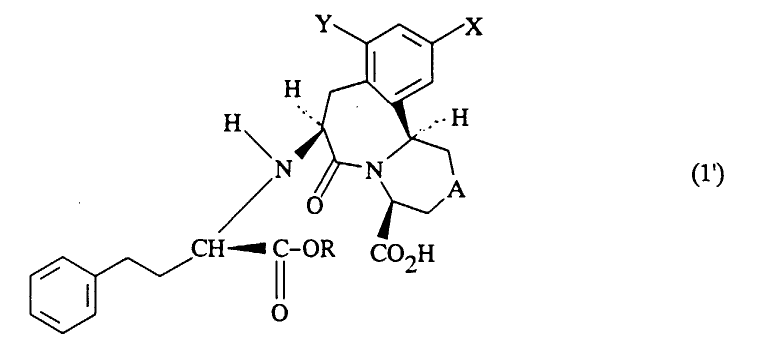 où
A est un radical méthylène, oxygène, soufre ou N-B où B est R₁ ou COR₂ où R₁ est un alkyle en C₁-C₄ ou un groupe Ar-Z où Ar est un aryle et Z est un alkyle en C₀-C₄ et R₂ est un radical -CF₃, un alkyle en C₁-C₁₀ ou un groupe Ar-Z ;
R est un hydrogène ; et
X et Y sont chacun indépendamment un radical hydrogène ou azote, pour autant que soit X soit Y soit un hydrogène et que soit X soit Y ne soit pas un hydrogène ; et
ses sels pharmaceutiquement acceptables,
comprenant la mise à réagir d'un composé de formule : 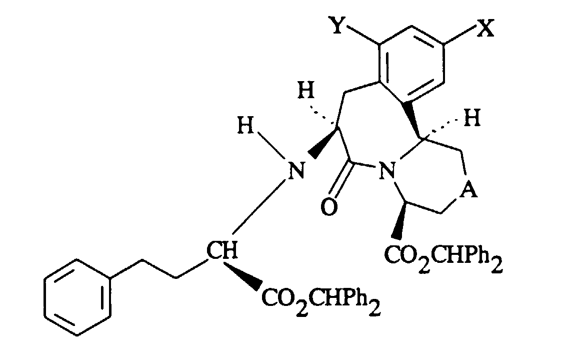 où Ph est un radical phényle avec un acide ou un agent réducteur.

17. Procédé de préparation d'un composé selon l'une quelconque des revendications 1 à 10, de formule 1b: 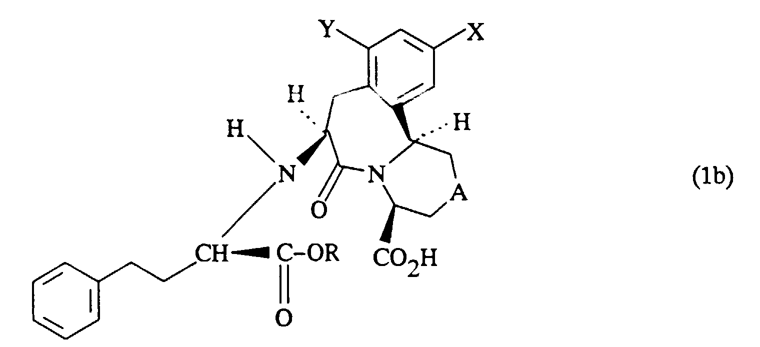 où
A est un radical méthylène, oxygène, soufre ou N-B où B est R₁ ou COR₂ où R₁ est un alkyle en C₁-C₄ ou un groupe Ar-Z où Ar est un aryle et Z est un alkyle en C₀-C₄ et R₂ est un radical -CF₃, un alkyle en C₁-C₁₀ ou un groupe Ar-Z ;
R est un alkyle en C₁-C₄ ; et
X et Y sont chacun indépendamment un radical hydrogène ou azote, pour autant que soit X soit Y soit un hydrogène et que soit X soit Y ne soit pas un hydrogène ; et
ses sels pharmaceutiquement acceptables,
comprenant la mise à réagir d'un composé de formule : 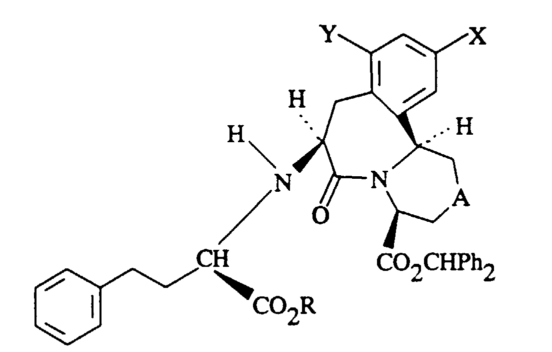 où Ph est un radical phényle avec un acide ou un agent réducteur.

18. Procédé de préparation d'un composé selon l'une quelconque des revendications 1 à 10, de formule 1c : 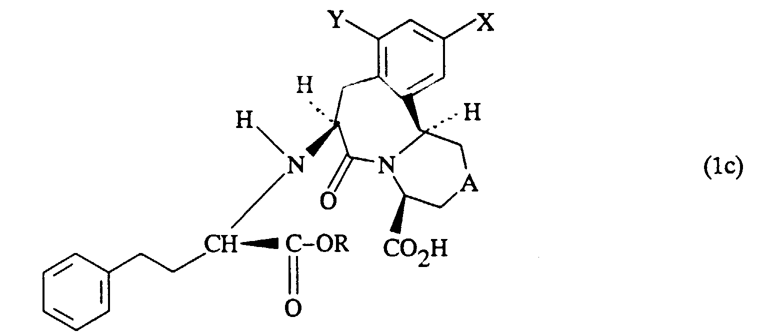 où
A est N-H ;
R est un hydrogène ou un alkyle en C₁-C₄ ; et
X et Y sont chacun indépendamment un hydrogène, un azote ou une amine, pour autant que soit X soit Y soit un hydrogène et soit X soit Y ne soit pas un hydrogène ; et
ses sels pharmaceutiquement acceptables,
comprenant la mise à réagir d'un composé de formule : 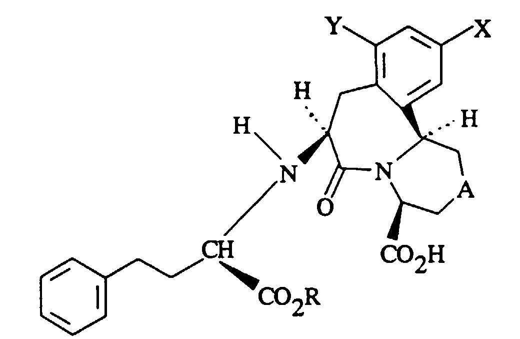 où A est N-COR₂ où R₂ est un radical -CF₃, un alkyle en C₁-C₁₀ ou un groupe Ar-Z où Ar est un aryle et Z est un alkyle en C₀-C₄ avec une base.

19. Procédé de préparation d'une composition pharmaceutique selon l'une quelconque des revendications il à 14, comprenant la combinaison d'un composé selon l'une quelconque des revendications 1 à 10 avec un ou plusieurs véhicules ou excipients pharmaceutiquement acceptables.
